# FASCICULE DE BREVET EUROPEEN

(11) **EP 3 727 389 B1**
(45) Date de publication et mention de la délivrance du brevet: **03.05.2023**
(21) Numéro de dépôt: 18826024.4
(22) Date de dépôt: 20.12.2018
(51) Int. Cl.: A61K 31/519, C07D 487/04

(54) **COMPOSES INHIBITEURS DE MTOR**
VERBINDUNGEN ALS MTOR-INHIBITOREN
COMPOUNDS AS MTOR INHIBITORS

(30) Priorité: 21.12.2017 FR 1771407
(43) Date de publication de la demande: 28.10.2020
(73) Titulaire: Galderma Research & Development, 06410 Biot (FR)
(72) Inventeur: CLARY, Laurence, 06480 La Colle sur Loup (FR); FOURNIER, Jean-François, 06600 Antibes (FR); OUVRY, Gilles, Abingdon, OXI143TA Oxfordshire (FR); BHURRUTH-ALCOR, Yushma, Ashburn, Virginie 20147 (US); THOREAU, Etienne, 06460 Saint Vallier de Thiey (FR); TOMAS, Loïc, 1814 La-Tour-de-Peilz (CH)
(74) Mandataire: Nederlandsch Octrooibureau
(86) Numéro de dépôt international: PCT/EP2018/086066
(87) Numéro de publication internationale: WO 2019/122059

(56) Documents cités:
- WO-A1-2012/148540
- WO-A2-2005/028434
- WO-A2-2009/062118

## Description

La présente invention concerne de nouveaux composés inhibiteurs de la sérine/thréonine kinase mTOR (« mechanistic target of rapamycin » ou cible fonctionnelle de la rapamycine, également connue sous le nom de FRAP, RAFT, RAPT, SEP). Elle concerne également des compositions les comprenant, leurs procédés de préparation et leurs utilisations dans des compositions en tant que médicament.

La protéine kinase mTOR est le centre catalytique de deux complexes multiprotéiques fonctionnellement distincts, conservés chez tous les eucaryotes et nommés mTORC1 et mTORC2 (Dunlop et al., « Mammalian target of rapamycin complex 1: signalling inputs, substrates and feedback mechanisms », 2009 ; Guertin et al., « The pharmacology of mTOR inhibition », 2009). Lorsqu'elle est associée à Raptor (regulatory associated protein of TOR) et à mLST8 (mammalian lethal with sec13 protein 8), mTOR forme le complexe mTORC1. Ce complexe interagit avec Deptor (DEP domaincontaining mTOR-interacting protein), FKBP38 et PRAS40 (prolinerich Akt substrate of 40 kDa), régulateurs négatifs de mTORC1. Pour former mTORC2, mTOR interagit avec les protéines Rictor (rapamycininsensitive companion of TOR), Sin1 (stress-activated map kinaseinteracting protein 1) et mLST8. De plus, mTORC2 s'associe aussi avec Deptor, qui réprime son activité, ainsi qu'avec PPR5/Protor, dont la fonction demeure inconnue. Lorsqu'elle est liée à FKBP12, la rapamycine inhibe spécifiquement mTORC1.

mTOR est notamment connue pour réguler la prolifération cellulaire, la croissance cellulaire, la mobilité cellulaire, la survie cellulaire, la biosynthèse des protéines et la transcription.

Il a été montré que les perturbations de la voie de signalisation de mTOR sont à l'origine de plusieurs maladies, en particulier différents types de cancer et des hamartomes multiples.

On connaît par exemple le document brevet WO2007061737 qui divulgue des composés bicycliques inhibiteurs de mTOR. Ils sont utilisés dans le traitement du cancer tel que le cancer du sein, cancer du poumon, cancer du poumon non à petites cellules, cancer du rein, carcinome rénal, cancer de la prostate, cancer du sang, cancer du foie, cancer de l'ovaire, cancer de la thyroïde, cancer de l'endomètre, lymphome, carcinome des cellules rénales, ou encore lymphome du manteau.

On connaît également le document brevet WO20091 1 7482 qui décrit plus particulièrement des sels et autres polymorphes de composés bicycliques inhibiteurs de mTOR, également utilisés dans le traitement du cancer, du même type que ceux décrits dans WO2007061737.

WO2012148540 concerne également des inhibiteurs de mTOR, sous forme de composés pyrazolo[3,4-d]-pyrimidine.

La rapamycine, inhibiteur de mTOR, est connue depuis longtemps pour ses propriétés immunosuppressives. Elle a néanmoins montré un succès thérapeutique limité lorsqu'elle est administrée par voie systémique à des patients atteints de psoriasis. Aussi, des données récentes ont montré que la voie de signalisation mTOR est hyperactivée dans la peau psoriasique lésionnelle, ce qui pourrait contribuer à la maladie en interférant avec la maturation des kératinocytes. L'effet du traitement topique de la rapamycine dans un modèle de souris psoriasique induite par l'imiquimod a été étudié (Bürger et al., «Blocking mTOR Signalling with Rapamycin Améliorâtes Imiquimod-induced Psoriasis in Mice», 2017). L'analyse immunohistologique a révélé que la rapamycine non seulement empêchait l'activation de la voie de signalisation mTOR (niveaux de P-mTOR et de P-S6), mais presque normalisait l'expression des marqueurs de différenciation épidermique. En outre, l'afflux de cellules immunitaires innées dans les ganglions lymphatiques drainants a été partiellement réduit par le traitement à la rapamycine. Ces données soulignent le rôle de la voie de signalisation mTOR dans la pathogenèse du psoriasis, et soutiennent l'étude de l'inhibition topique de la mTOR en tant que nouvelle stratégie anti-psoriasique.

Il existe donc un réel besoin de développer des traitements en particulier topiques de patients atteints de maladies telles que le psoriasis.

Considérant ce qui précède, un problème que se propose de résoudre l'invention est de proposer de nouveaux inhibiteurs de mTOR pour améliorer notamment le traitement de maladies prolifératives ou inflammatoires à médiation immunitaire de la peau.

La Demanderesse a développé de nouveaux composés inhibiteurs de mTOR.

Ainsi, la présente invention a pour objet un composé inhibiteur de mTOR de formule générale (I) 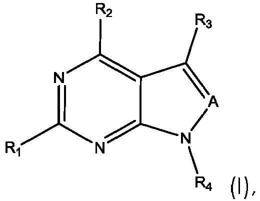 ou un de ses sels pharmaceutiquement acceptables, dans laquelle chaque variable est telle que définie et décrite ci-après.

L'invention a également pour objet une composition comprenant, dans un milieu physiologiquement acceptable, un composé inhibiteur de mTOR de formule (I) selon l'invention ou un de ses sels pharmaceutiquement acceptables. Elle est destinée à être utilisée en tant que médicament, en particulier dans le traitement des maladies impliquant une enzyme mTOR à activité sérine-thréonine kinase et notamment dans le traitement des affections dermatologiques liées à un trouble de la kératinisation avec une composante proliférative, inflammatoire et/ou immuno-allergique telles que le psoriasis, la dermatite atopique, la kératose actinique, ou l'acné, préférentiellement la dermatite atopique, plus préférentiellement la composante inflammatoire de la dermatite atopique et encore plus préférentiellement le traitement par voie topique de la composante inflammatoire de la dermatite atopique.

L'invention sera mieux comprise à la lecture de la description qui suit, rédigée au regard des dessins annexés, dans lesquels :
- la Figure 1 représente une voie de synthèse du composé 3-(2-amino-benzooxazol-5-yl)-1-((*S*)-1,3-dimethyl-butyl)-1*H*-pyrazolo[3,4-*d*]pyrimidine-4,6-diamine (exemple 1) ;
- la Figure 2 représente une voie de synthèse du composé 3-(2-Amino-benzooxazol-5-yl)-1-isopropyl-*N*⁶-methyl-1*H*-pyrazolo[3,4-*d*]pyrimidine-4,6-diamine (exemple 26) ;
- la Figure 3 représente une voie de synthèse du composé 2-[4,6-diamino-1-(2,2-dimethyl-propyl)-1*H*-pyrazolo[3,4-*d*]pyrimidin-3-yl]-1*H-*pyrrolo[2,3-*b*]pyridin-5-ol (exemple 19) ;
- la Figure 4 représente une voie de synthèse des composés 3-(2-amino-benzooxazol-5-yl)-4-chloro-1-isobutyl-1H-pyrazolo[3,4-d]pyrimidin-6-ylamine (exemple 23), 3-(2-amino-benzooxazol-5-yl)-1-isopropyl-1H-pyrazolo[3,4-d]pyrimidin-6-ylamine (exemple 24) et 5-(6-amino-4-methyl-1-neopentyl-1H-pyrazolo[3,4-d]pyrimidin-3-yl)benzo[d]oxazol-2-amine (exemple 27) ;
- la Figure 5 représente une voie de synthèse du composé 5-(6-Amino-4-chloro-1-isopropyl-1H-pyrazolo[3,4-d]pyrimidin-3-yl)benzo[d]oxazol-2-amine (exemple 31) ;
- la Figure 6 représente un chronogramme d'un modèle de patch de dermatite atopique chez la souris selon l'exemple 33 ;
- la Figure 7 représente les résultats de test de perte d'eau transépidermique (ou TEWL) dans un modèle de patch de dermatite atopique après application d'un antagoniste de mTOR selon l'exemple 34 ;
- la Figure 8 représente l'épaisseur de l'épiderme dans un modèle de patch de dermatite atopique après application d'un antagoniste de mTOR selon l'exemple 35 ; et
- la Figure 9 représente le score d'inflammation dans un modèle de patch de dermatite atopique après application d'un antagoniste de mTOR selon l'exemple 36.

La présente invention concerne des nouveaux composés inhibiteurs de mTOR ou un de leurs sels pharmaceutiquement acceptables.

Par inhibiteur de mTOR, on entend des composés qui vont réguler négativement c'est-à-dire réduire, bloquer voire supprimer l'activation de la voie de signalisation mTOR, en faisant compétition, avantageusement de manière sélective, avec les substrats au niveau de mTORC1 et/ou mTORC2 ou en modifiant le site actif de ces enzymes qui ne peuvent ainsi plus catalyser un substrat donné. On utilise indifféremment les termes antagoniste (de mTOR) ou inhibiteur (de mTOR) selon la présente invention.

Les composés selon l'invention peuvent être représentés par la formule générale (I) 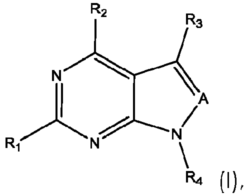 ou un de leurs sels pharmaceutiquement acceptables, dans laquelle :
R₁ représente un radical -NH₂, -NHMe, -NHEt ;
R₂ représente un atome d'hydrogène, un atome d'halogène choisi parmi F, CI, Br et I, un radical -NH₂, -NHalkyle, -NHAc, nitrile, méthyle (Me), éthyle (Et), trifluorométhyle, -OH, méthoxy ;
étant entendu que lorsque R₂ est différent d'un radical -NH₂, alors R₁ représente un radical -NH₂;
R₃ représente un radical aromatique ou hétéroaromatique simple ou bicycle fusionné, non substitué ou mono- ou polysubstitué par un ou plusieurs radical(aux) choisi(s) parmi un atome d'halogène choisi parmi F, CI, Br et I, - NHRs, nitrile, méthyle, éthyle, trifluorométhyle, -OR₆ ;
   avec R₅ représentant un atome d'hydrogène, un radical cyclopropyle, acyle, alkyle en C₁-C₆ saturé ou insaturé, interrompu ou non par un hétéroatome O, S, et non substitué ou substitué par un cycloalkyle ou un hétérocycloalkyle en C₃-C₅ ; et
   avec R₆ représentant un atome d'hydrogène ou un radical méthyle ;
R₄ représente un atome d'hydrogène, un radical alkyle en C₁-C₁₀ linéaire ou ramifié, un cycle ou bicycle en C₃-C₁₀ saturé ou insaturé, interrompu ou non par un ou plusieurs hétéroatomes O, S et N, et non substitué ou substitué par un radical sulfone, fluoro, cyano, ester, -NR₇, -NR₇R₈, cycloalkyle ou hétérocycloalkyle en C₃-C₆, ou un cycle aromatique ou un hétérocycle non substitué ou mono- ou poly-substitué par un atome d'halogène choisi parmi CI, F ou un radical -OH, -OMe, trifluorométhyle, méthyle, éthyle, -NH₂, -NHMe, -NMe₂.
   avec R₇, R₈ représentant, indépendamment l'un de l'autre, un atome d'hydrogène, un radical alkyle en C₁-C₃, cyclopropyle ou acyle, ou formant ensemble un cycle en C₃-C₅ ; et
A représente un radical -CH ou un atome d'azote.

Selon la présente invention, par alkyle, on entend un radical linéaire ou ramifié ayant par exemple de 1 à 6 (en C₁-C₆), ou de 1 à 3 (en C₁-C₃), atomes de carbone, avantageusement les radicaux méthyle, éthyle, propyle, isopropyle, butyle, tertiobutyle, 2-méthylbutyle, pentyle, 2-méthylpentyle, hexyle.

Par acyle, on entend un radical obtenu en enlevant le groupement hydroxyle d'un acide carboxylique ; le groupement acyle correspondant à un acide carboxylique de formule -RCOOH aura pour formule -RCO, où l'atome de carbone et celui d'oxygène sont liés par une double liaison (groupement carbonyle).

Par cycloalkyle, on entend un radical cycloalkyle ayant de 3 à 6 atomes de carbone avantageusement choisi parmi cyclopropyle, cyclobutyle, cyclopentyle, cyclohexyle.

Par hétérocycloalkyle ou hétérocycle, on entend par exemple un radical pipéridino, morpholino, pyrrolidino ou pipérazino.

Par cycle aromatique, on entend un radical cyclique plan et possédant (4n + 2) électrons délocalisés, n étant le nombre de cycles constituant le radical ; si le cycle contient des éléments autres que le carbone et l'hydrogène, on parle d'hétérocycle aromatique ou de groupement hétéroaryle.

Lorsque les composés selon l'invention se présentent sous forme d'un sel pharmaceutiquement acceptable, il s'agit de préférence d'un sel obtenu à partir d'une base ou d'un acide non toxiques.

Le terme « sel pharmaceutiquement acceptable » se réfère aux sels qui sont, dans le cadre d'un bon jugement médical, appropriés pour une utilisation en contact avec les tissus humains et animaux inférieurs sans toxicité excessive, irritation, réponse allergiques et similaires et sont proportionnels à un rapport bénéfice/risque raisonnable. Les sels pharmaceutiquement acceptables sont bien connus dans l'état de l'art. Les sels pharmaceutiquement acceptables des composés de la présente invention comprennent ceux dérivés d'acides et de bases inorganiques et organiques appropriés.

Lorsque le composé de la présente invention est acide, son sel correspondant peut être préparé à partir de bases non toxiques pharmaceutiquement acceptables, comprenant des bases inorganiques et des bases organiques.

Les sels dérivés de ces bases inorganiques comprennent l'aluminium, l'ammonium, le calcium, le cuivre, le fer, le lithium, le magnésium, le manganèse, le potassium, le sodium et les sels similaires. Les sels d'ammonium, de calcium, de magnésium, de potassium et de sodium sont particulièrement préférés.

Les sels dérivés de bases organiques non toxiques pharmaceutiquement acceptables comprennent des sels d'amines primaires, secondaires et tertiaires, ainsi que des amines cycliques et des amines substituées telles que des amines substituées naturellement et synthétisées.

D'autres bases organiques non toxiques pharmaceutiquement acceptables à partir desquelles des sels peuvent être formés comprennent des résines échangeuses d'ions telles que, par exemple, l'arginine, bétaïne, caféine, choline, N',N'-dibenzyléthylènediamine, diéthylamine, 2-diéthylaminoéthanol, 2-diméthylaminoéthanol, éthanolamine, N,N-diéthyléthanolamine, éthylènediamine, N-éthylmorpholine, N-éthylpipéridine, glucamine, glucosamine, histidine, hydrabamine, isopropylamine, lysine, méthylglucamine, morpholine, pipérazine, pipéridine, résines polyamine, procaïne, purines, théobromine, triéthylamine, triméthylamine, tripropylamine, trométhamine et similaires.

Lorsque le composé de la présente invention est basique, son sel correspondant peut être préparé à partir d'acides non toxiques pharmaceutiquement acceptables, comprenant des acides inorganiques et organiques.

De tels acides comprennent, par exemple, un acide acétique, benzènesulfonique, benzoïque, camphorsulfonique, citrique, éthanesulfonique, fumarique, gluconique, glutamique, bromhydrique, chlorhydrique, iséthionique, lactique, maléique, malique, mandélique, méthanesulfonique, mucique, nitrique, pamoïque, pantothénique, phosphorique, succinique, sulfurique, tartrique, p-toluènesulfonique et similaires. Les acides citrique, bromhydrique, chlorhydrique, maléique, phosphorique, sulfurique et tartrique sont particulièrement préférés.

Selon un mode de réalisation de l'invention, le sel pharmaceutiquement acceptable est choisi parmi la trométhamine, le sodium, le calcium et la L-arginine.

Selon un autre mode de réalisation de l'invention, le sel est choisi parmi le magnésium, potassium, N,N-diéthyléthanolamine, N-méthyle-D-glucamine ou pipérazine.

Dans certains modes de réalisation, le sel est sous forme de sel d'hydrate ou de solvate.

Dans certains modes de réalisation, le sel est sensiblement sous forme amorphe.

Dans certains modes de réalisation, le sel est essentiellement sous forme cristalline.

Dans certains modes de réalisation, le sel est au moins à environ 95% en poids cristallin.

Dans certains modes de réalisation, le sel est sensiblement sous forme cristalline unique.

Selon la présente invention, les composés préférés sont ceux de formule générale (I) 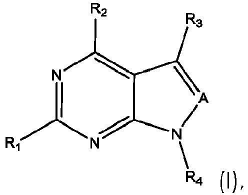 ou un de leurs sels pharmaceutiquement acceptables, dans laquelle :
R₁ représente un radical -NH₂ ;
R₂ représente un atome d'halogène choisi parmi F, CI, Br et I, ou un radical -NH₂ ;
R₃ représente un radical hétéroaromatique bicycle fusionné, non substitué ou mono- ou polysubstitué par un ou plusieurs radical(aux) choisi(s) parmi un atome d'halogène choisi parmi F, Cl, Br et I, -NHR₅, nitrile, méthyle, éthyle, trifluorométhyle, -OR₆ ;
   avec R₅ représentant un atome d'hydrogène, un radical cyclopropyle, acyle, alkyle en C₁-C₆ saturé ou insaturé, interrompu ou non par un hétéroatome O, S, et non substitué ou substitué par un cycloalkyle ou un hétérocycloalkyle en C₃-C₅ ; et
   avec R₆ représentant un atome d'hydrogène ou un radical méthyle ;
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆ ; ; et
A représente un atome d'azote.

Les composés plus préférés selon l'invention sont ceux de formule générale (I), ou un de leurs sels pharmaceutiquement acceptables, dans laquelle :
R₁ représente un radical -NH₂ ;
R₂ représente un atome CI ou un radical -NH₂ ;
R₃ représente un radical hétéroaromatique bicycle fusionné, mono- ou polysubstitué par un ou plusieurs radical(aux) choisi(s) parmi un atome d'halogène choisi parmi F, CI, Br et I, -NHR₅, nitrile, méthyle, éthyle, trifluorométhyle, -OR₆ ;
   avec R₅ représentant un atome d'hydrogène, un radical cyclopropyle, acyle, alkyle en C₁-C₆ saturé ou insaturé, interrompu ou non par un hétéroatome O, S, et non substitué ou substitué par un cycloalkyle ou un hétérocycloalkyle en C₃-C₅ ; et
   avec R₆ représentant un atome d'hydrogène ou un radical méthyle ;
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆ ; et
A représente un atome d'azote.

Selon la présente invention, les composés encore plus préférés sont ceux de formule (Ia), 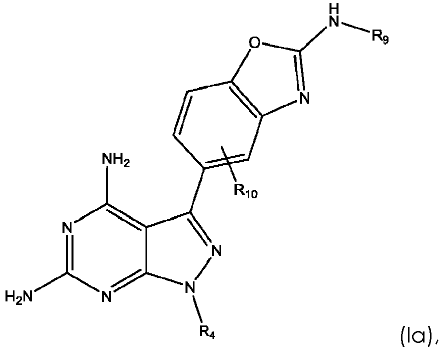 ou un de leurs sels pharmaceutiquement acceptables, dans laquelle :
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆ ;
R₉ représente un atome d'hydrogène, ou un radical méthyle, éthyle ; et
un ou plusieurs R₁₀, pris indépendamment, représentent un atome d'hydrogène, un atome d'halogène choisi parmi Cl, F, ou un radical -OH, méthyle, -OMe.

Plus préférentiellement, les composés de formule (I) ou (Ia), ou un de leurs sels pharmaceutiquement acceptables, sont ceux dans lesquelles :
R₄ représente un alkyle en C₃-C₁₀ ramifié ;
R₉ représente un atome d'hydrogène ; et
un ou plusieurs R₁₀, pris indépendamment, représentent un atome d'hydrogène, un atome d'halogène choisi parmi Cl, F, ou un radical méthyle.

Encore plus préférentiellement, les composés de formule (I) ou (la), ou un de leurs sels pharmaceutiquement acceptables, sont ceux dans lesquelles :
R₄ représente un alkyle en C₄-C₆ ramifié ;
R₉ représente un atome d'hydrogène ; et
R₁₀ représente un atome d'hydrogène ou de fluor.

Le composé particulièrement préféré de formule (I) ou (la), ou un de ses sels pharmaceutiquement acceptables, sont ceux dans lesquelles :
R₄ représente un radical (S)-1,3-diméthyle-butyle ;
R₉ représente un atome d'hydrogène ; et
R₁₀ représente un atome d'hydrogène.

Parmi les composés de formule (I) ou (la) entrant dans le cadre de la présente invention, on peut notamment citer les composés suivants :
- 3-(2-amino-benzooxazol-5-yl)-1-((S)-1,3-dimethyl-butyl)-1H-pyrazolo[3,4-d]pyrimidine-4,6-diamine;
- 3-(2-amino-6-fluoro-benzooxazol-5-yl)-1-((S)-1,3-dimethyl-butyl)-1H-pyrazolo[3,4-d]pyrimidine-4,6-diamine;
- 3-(2-amino-4-fluoro-benzooxazol-5-yl)-1-(1-methyl-cyclobutylmethyl)-1H-pyrazolo[3,4-d]pyrimidine-4,6-diamine;
- 3-(2-amino-6-fluoro-benzooxazol-5-yl)-1-(1-methyl-cyclobutylmethyl)-1H-pyrazolo[3,4-d]pyrimidine-4,6-diamine;
- 3-(2-amino-4-fluoro-benzooxazol-5-yl)-1-((S)-1,3-dimethyl-butyl)-1H-pyrazolo[3,4-d]pyrimidine-4,6-diamine;
- N-(5-(4,6-diamino-1-(2,2-dimethylbutyl)-1H-pyrazolo[3,4-d] pyri midin-3-yl) benzo [d] oxazol-2-yl)acetamide;
- 3-(2-amino-benzooxazol-5-yl)-1-(2,2-dimethyl-propyl)-1H-pyrazolo[3,4-d]pyrimidine-4,6-diamine;
- 3-(2-amino-benzooxazol-5-yl)-1-(2,2-dimethyl-butyl)-1H-pyrazolo[3,4-d]pyrimidine-4,6-diamine;
- 3-(2-amino-benzooxazol-5-yl)-1-(3-methyl-thietan-3-ylmethyl)-1H-pyrazolo[3,4-d]pyrimidine-4,6-diamine;
- 3-(2-amino-4-fluoro-benzooxazol-5-yl)-1-(2,2-dimethyl-butyl)-1H-pyrazolo[3,4-d]pyrimidine-4,6-diamine;
- 1-(2,2-dimethyl-propyl)-3-(2-ethylamino-benzooxazol-5-yl)-1H-pyrazolo[3,4-d]pyrimidine-4,6-diamine;
- 3-(2-amino-benzooxazol-5-yl)-1-(2,2-dimethyl-but-3-enyl)-1H-pyrazolo[3,4-d]pyrimidine-4,6-diamine;
- (+)-3-(2-amino-benzooxazol-5-yl)-1-((S)-2-methyl-2-tetrahydro-furan-2-yl-propyl)-1H-pyrazolo[3,4-d]pyrimidine-4,6-diamine;
- 3-(2-amino-benzooxazol-5-yl)-1-(2,2-dimethyl-but-3-ynyl)-1H-pyrazolo[3,4-d]pyrimidine-4,6-diamine;
- 3-[4,6-diamino-3-(2-amino-benzooxazol-5-yl)-pyrazolo[3,4-d]pyrimidin-1-yl]-2,2-dimethyl-propan-1-ol;
- 3-(2-amino-benzooxazol-5-yl)-1-(2-methyl-2-methylsulfanyl-propyl)-1H-pyrazolo[3,4-d]pyrimidine-4,6-diamine;
- 3-(2-amino-4-chloro-benzooxazol-5-yl)-1-(2,2-dimethyl-propyl)-1H-pyrazolo[3,4-d]pyrimidine-4,6-diamine;
- 3-(2-amino-6-methoxy-benzooxazol-5-yl)-1-(2,2-dimethyl-propyl)-1H-pyrazolo[3,4-d]pyrimidine-4,6-diamine;
- 2-[4,6-diamino-1-(2,2-dimethyl-propyl)-1H-pyrazolo[3,4-d]pyrimidin-3-yl]-1H-pyrrolo[2,3-b]pyridin-5-ol;
- 3-(2-amino-6-methoxy-benzooxazol-5-yl)-1-(2,2-dimethyl-butyl)-1H-pyrazolo[3,4-d]pyrimidine-4,6-diamine;
- 3-(2-amino-benzooxazol-5-yl)-1-isopropyl-1H-pyrazolo[3,4-d]pyrimidine-4,6-diamine;
- 3-(2-amino-benzooxazol-5-yl)-1-(2-ethyl-2-methanesulfonyl-butyl)-1H-pyrazolo[3,4-d]pyrimidine-4,6-diamine;
- 3-(2-amino-benzooxazol-5-yl)-4-chloro-1-isobutyl-1H-pyrazolo[3,4-d]pyrimidin-6-ylamine;
- 3-(2-amino-benzooxazol-5-yl)-1-isopropyl-1H-pyrazolo[3,4-d]pyrimidin-6-ylamine;
- 3-(2-amino-6-chloro-benzooxazol-5-yl)-1-((S)-1,2-dimethyl-propyl)-1H-pyrazolo[3,4-d]pyrimidine-4,6-diamine;
- 3-(2-Amino-benzooxazol-5-yl)-1-isopropyl-N^{∗}6^{∗}-methyl-1H-pyrazolo[3,4-d]pyrimidine-4,6-diamine;
- 5-(6-amino-4-methyl-1-neopentyl-1H-pyrazolo[3,4-d]pyrimidin-3-yl)benzo[d]oxazol-2-amine;
- 3-(2-cyclopropylamino-benzooxazol-5-yl)-1-isopropyl-1H-pyrazolo[3,4-d]pyrimidine-4,6-diamine;
- 3-(2-amino-7-methyl-benzooxazol-5-yl)-1-(2,2-dimethyl-butyl)-1H-pyrazolo[3,4-d]pyrimidine-4,6-diamine;
- 3-(2-amino-benzooxazol-5-yl)-1-(1-methyl-cyclobutylmethyl)-1H-pyrazolo[3,4-d]pyrimidine-4,6-diamine;
- 5-(6-Amino-4-chloro-1-isopropyl-1*H*-pyrazolo[3,4-d]pyrimidin-3-yl)benzo[d]oxazol-2-amine.

La présente invention a également pour objet une composition comprenant, dans un milieu physiologiquement acceptable, un composé de formule (I) ou (Ia) selon l'invention tel que défini ci-avant ou un de ses sels pharmaceutiquement acceptables.

Un milieu pharmaceutiquement acceptable désigne un milieu compatible et adapté pour une utilisation en contact avec des cellules humaines et animales, en particulier avec la peau, les muqueuses et/ou les phanères, sans toxicité, irritation, réponse allergique indue et similaires, et proportionné à un rapport avantage/risque raisonnable.

Un milieu pharmaceutiquement acceptable selon l'invention peut comprendre tout adjuvant connu et utilisé dans le domaine pharmaceutique, compatible avec les composés inhibiteurs de mTOR selon l'invention.

A titre d'exemple on peut citer des solvants, tampons, aromatisants, liants, agents chélatants, agents tensioactifs, épaississants, lubrifiants, gélifiants, humectants, hydratants, conservateurs, antioxydants, agents apaisants, agents pro-pénétrants, colorants, parfums et similaires, ou leur mélange.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée. Leur concentration est également choisie de sorte à ce qu'elle ne nuise pas aux propriétés avantageuses des composés selon l'invention.

Le composé selon la présente invention, et la composition le comprenant, peuvent être administrés par voie orale, rectale, topique, ou parentérale (sous-cutanée, intramusculaire ou intraveineuse). Ils sont de préférence administrés par voie orale ou topique, plus préférentiellement topique.

Les compositions selon l'invention peuvent se présenter sous forme liquide, solide ou encore de gaz.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie parentérale, la composition peut se présenter sous forme de solutions ou suspensions pour perfusion ou pour injection.

De préférence, la composition pharmaceutique est conditionnée sous une forme convenant à une application par voie topique.

Par voie topique, les compositions, qui sont donc plus particulièrement destinées au traitement de la peau et des muqueuses, peuvent se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de lotions, de suspensions, de sticks, de shampoings, ou de bases lavantes. Elle peut également se présenter sous forme de suspensions de microsphères ou nanosphères, de vésicules lipidiques ou polymériques, de patches polymériques ou gélifiés, ou d'hydrogels permettant une libération contrôlée des composés actifs. Ces compositions par voie topique peuvent par ailleurs se présenter soit sous forme anhydre, soit sous une forme aqueuse.

La composition selon l'invention comprend préférentiellement entre 0,001% et 5% dudit composé de formule (I) ou (Ia) ou un de ses sels pharmaceutiquement acceptables, en poids du poids total de la composition.

La quantité réellement administrée à mettre en oeuvre selon l'invention dépend de l'effet thérapeutique recherché, et peut donc varier dans une large mesure. L'homme de l'art, en particulier le médecin peut aisément, sur la base de ses connaissances générales déterminer les quantités appropriées.

La composition selon l'invention peut comprendre au moins un autre ingrédient actif.

L'ingrédient actif additionnel est préférentiellement choisi parmi le groupe comprenant les antibiotiques, antibactériens, antiviraux, antiparasitaires, antifongiques, anesthésiques, analgésiques, antiallergiques, rétinoïdes, anti-radicaux libres, antiprurigineux, antihistaminiques, immunosuppresseurs, corticostéroïdes, agents kératolytiques, immunoglobulines intraveineuses, anti-angiogéniques, anti-inflammatoires et/ou un mélange de ceux-ci.

Plus préférentiellement, l'ingrédient actif additionnel est connu pour son efficacité dans le traitement des affections dermatologiques liées à un trouble de la kératinisation avec une composante proliférative, inflammatoire et/ou immuno-allergique telles que le psoriasis, la dermatite atopique, la kératose actinique, ou l'acné.

La présente invention concerne de nouveaux composés inhibiteurs de mTOR de formule (I) ou (Ia).

Ainsi, la présente invention a pour objet les composés de formule (I) ou (Ia) tels que décrits ci-dessus destinés à être utilisés à titre de médicament.

L'invention a également pour objet une composition selon l'invention, pour son utilisation en tant que médicament, en particulier dans le traitement des maladies impliquant une enzyme mTOR à activité sérine-thréonine kinase chez un patient.

Les termes « traiter » ou « traitement », tels qu'utilisés dans la présente invention, se rapportent à l'inversion, à l'atténuation, à l'inhibition de la progression, au retard de l'apparition, à l'amélioration et/ou ou soulagement partiel ou total d'une maladie ou d'un trouble, ou d'un ou plusieurs symptômes de la maladie ou du trouble, tels que décrits ci-après. Dans certains modes de réalisation, le traitement peut être administré après qu'un ou plusieurs symptômes se sont développés. Dans certains modes de réalisation particuliers, le traitement peut être administré en tant que mesure préventive, pour prévenir ou arrêter la progression d'une maladie ou d'un trouble. Dans ce contexte, la « prévention » désigne une réduction du risque d'acquisition d'une maladie ou d'un trouble déterminé. Dans d'autres modes de réalisation, le traitement peut être administré en l'absence de symptômes. Par exemple, le traitement peut être administré à un individu prédisposé avant l'apparition des symptômes (par exemple à la lumière d'antécédents de symptômes et/ou de facteurs génétiques ou autres facteurs de prédisposition). Le traitement peut également être poursuivi après la disparition des symptômes, par exemple pour prévenir ou retarder leur réapparition. Ainsi, dans certains modes de réalisation, le terme « traitement » comprend la prévention d'une rechute ou d'une récurrence d'une maladie ou d'un trouble.

Le terme « patient », tel qu'utilisé dans la présente invention, désigne un mammifère et inclut des sujets humains et animaux, de préférence un humain.

La composition selon l'invention est plus particulièrement destinée à être utilisée dans le traitement des affections dermatologiques liées à un trouble de la kératinisation avec une composante proliférative, inflammatoire et/ou immuno-allergique.

Les affections dermatologiques liées à un trouble de la kératinisation avec une composante proliférative, inflammatoire et/ou immuno-allergique comprennent les troubles ou désordres de la kératinisation portant sur la prolifération cellulaire notamment, les acnés vulgaires, comédoniennes, polymorphes, rosacées, les acnés nodulokystiques, conglobata, les acnés séniles, les acnés secondaires telles que l'acné solaire, médicamenteuse ou professionnelle, les autres troubles de la kératinisation, notamment les ichtyoses, les états ichtyosif ormes, la maladie de Darier, les keratodermies palmoplantaires, les leucoplasies et les états leucoplasiformes, le lichen cutané ou muqueux (buccal), les autres affections dermatologiques liées à un trouble de la kératinisation avec une composante inflammatoire et/ou immuno-allergique notamment, toutes les formes de psoriasis qu'il soit cutané, muqueux ou unguéal, et même le rhumatisme psoriatique, ou encore l'atopie cutanée, telle que la dermatite atopique (ou eczéma atopique) ou l'atopie respiratoire ou encore l'hypertrophie gingivale, toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les lésions ou proliférations pouvant être induites par les ultra-violets notamment dans le cas des kératoses actiniques, épithélioma baso et spinocellulaires.

Plus préférentiellement, la composition selon l'invention est destinée à être utilisée dans le traitement des affections dermatologiques liées à un trouble de la kératinisation avec une composante proliférative, inflammatoire et/ou immuno-allergique telles que le psoriasis, la dermatite atopique, la kératose actinique, ou l'acné, encore plus préférentiellement la dermatite atopique.

De manière particulièrement avantageuse, la composition selon l'invention est destinée à être utilisée dans le traitement de la composante inflammatoire de la dermatite atopique, et préférentiellement le traitement par voie topique de la composante inflammatoire de la dermatite atopique.

Selon un autre mode particulièrement avantageux de l'invention, la composition est destinée à être utilisée pour renforcer la fonction de barrière cutanée chez un patient souffrant de dermatite atopique.

Par « composante inflammatoire de la dermatite atopique », on entend une inflammation impliquant des lymphocytes CD4+, des éosinophiles, des mastocytes et des cytokines Th2.

On entend par « fonction barrière » ou « barrière cutanée » le rôle protecteur des cellules épidermiques, en particulier de la couche cornée, vis-à-vis de l'environnement (i.e. perte d'eau, agressions physiques et/ou chimiques et agents infectieux).

La fonction barrière peut être évaluée à l'aide du test de perte d'eau trans-épidermique (ou TEWL) et/ou de l'évaluation histologique de l'épaisseur de l'épiderme. Par perte d'eau trans-épidermique, on entend le pourcentage d'eau passant à travers les matières kératiniques (plus précisément la couche cornée) et s'évaporant à leur surface. Le protocole de mesure du TEWL est détaillé dans les exemples ci-après.

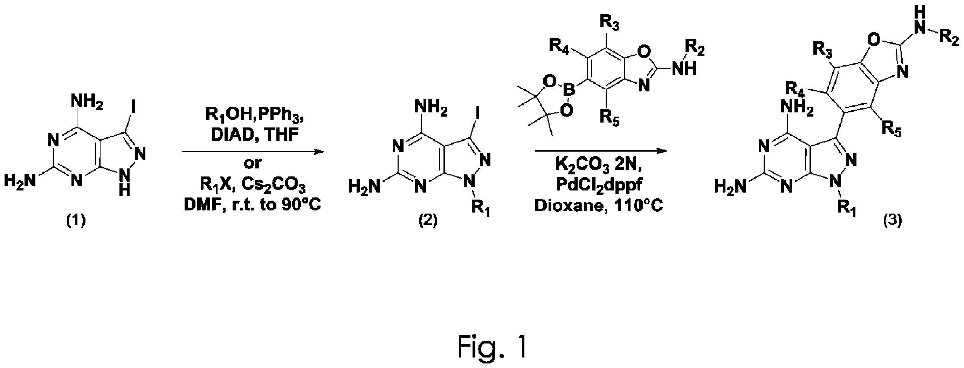

La présente invention a également pour objet les procédés de préparation des composés de formule (I) ou (Ia), en particulier selon le schéma réactionnel donné à la Figure 1.

On va maintenant donner, à titre d'illustration plusieurs exemples d'obtention de composés actifs de formule (I) ou (Ia) selon l'invention et des résultats d'activité inhibitrice.

Exemple 1 : voie de synthèse du composé 3-(2-amino-benzooxazol-5-yl)-1-((S)-1,3-dimethyl-butyl)-1*H*-pyrazolo[3,4-*d*] pyrimidine-4,6-diamine (exemple 1) telle qu'illustrée à la Figure 1

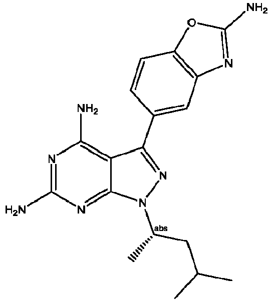

### a) 1-((S)-1,3-dimethyl-butyl)-3-iodo-1H-pyrazolo[3,4-d]pyrimidine-4,6-diamine

Du (*R*)-4-methyl-pentan-2-ol (0.25 ml; 2.0 mmol; 1.1 eq.) est ajouté sur du 3-iodo-1*H*-pyrazolo[3,4-*d*]pyrimidine-4,6-diamine (500 mg; 1.8 mmol; 1.0 eq.) (1) et de triphénylphosphine (1.43 g; 5.4 mmol; 3.0 eq.) en solution dans du tétrahydrofurane (10 ml). Le milieu réactionnel est refroidi à 0°C puis du diisopropylazodicarboxylate (1.1 ml; 5.4 mmol; 3.0 eq.) est ajouté. Le milieu réactionnel est agité à température ambiante pendant 30 minutes. La réaction est arrêtée par addition d'eau et d'une solution de soude 1N à froid jusqu'à pH basique puis est extraite à l'acétate d'éthyle. La phase organique est lavée avec une solution saturée de chlorure de sodium, séchée sur sulfate de sodium et concentrée. Le produit brut est chromatographié sur gel de silice (25g, dépôt solide, éluant dichlorométhane/méthanol de 0 à 15% de méthanol). Le 1-((*S*)-1,3-dimethyl-butyl)-3-iodo-1*H*-pyrazolo[3,4-*d*]pyrimidine-4,6-diamine (308 mg; 47%) (2) est obtenu sous la forme d'une meringue jaune pâle.

### b) 3-(2-amino-benzooxazol-5-yl)-1-((S)-1,3-dimethyl-butyl)-1H-pyrazolo[3,4-d]pyrimidine-4,6-diamine

Une solution de 1-((S)-1,3-dimethyl-butyl)-3-iodo-1*H*-pyrazolo[3,4-*d*]pyrimidine-4,6-diamine (570 mg; 1.6 mmol; 1.0 eq.) (2), de dichlorhydrate d'acide (2-aminobenzo[d]oxazol-5-yl)boronique (407 mg; 1.9 mmol; 1.2 eq.) et une solution de carbonate de potassium (2.4 ml; 2.0 M; 4.8 mmol; 3.0 eq.) dans du 1,4-dioxane (5.7 ml) est dégazée sous azote pendant 10 minutes puis du 1,1'-bis(diphenylphosphino)ferrocenpalladium(II) dichloride, dichlorométhane (65 mg; 0.08 mmol; 0.05 eq.) est rajouté. Le milieu est chauffé à 1 10°C pendant 30 minutes. La réaction est arrêtée par addition d'eau puis extraite à l'acétate d'éthyle. Les phases organiques sont rassemblées, lavées avec une solution saturée de chlorure de sodium, séchées sur sulfate de sodium et concentrées. Le produit brut est chromatographié sur gel de silice (12 g, dépôt solide, éluant dichlorométhane/méthanol de 0 à 15% de méthanol, CCM : dichlorométhane/méthanol 95/5 Rf=0.07). Le 3-(2-amino-benzooxazol-5-yl)-1-((S)-1,3-dimethyl-butyl)-1*H*-pyrazolo[3,4-*d*]pyrimidine-4,6-diamine (399 mg; 69%) (3) est obtenu sous la forme d'un solide cristallin blanc après recristallisation dans acétonitrile/eau.

1H RMN (DMSO-d6) δ: 0.81 (d, J = 6.5 Hz, 3H), 0.89 (d, J = 6.5 Hz, 3H), 1.20 - 1.34 (m, 1H), 1.37 (d, J = 6.7 Hz, 3H), 1.52 (ddd, J = 13.6, 8.6, 5.1 Hz, 1H), 2.00 (ddd, J = 13.6, 10.0, 5.4 Hz, 1H), 4.72 - 4.82 (m, 1H), 6.11 (s, 4H), 7.19 (dd, J = 8.2, 1.8 Hz, 1H), 7.36 (d, J = 1.9 Hz, 1H), 7.44 (dd, J = 8.1, 5.2 Hz, 1H), 7.50 (s, 2H) MS (ESI) m/z = 367 [M+H]⁺

### Exemple 2 : 3-(2-amino-6-fluoro-benzooxazol-5-yl)-1-((S)-1,3-dimethyl-butyl)-1H-pyrazolo[3,4-d]pyrimidine-4,6-diamine

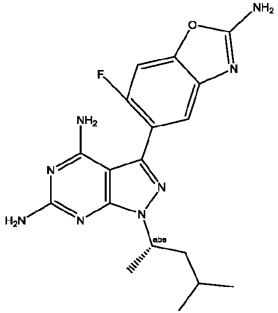

### a) 1-((S)-1,3-dimethyl-butyl)-3-iodo-1H-pyrazolo[3,4-d]pyrimidine-4,6-diamine

Du (R)-4-methyl-pentan-2-ol (0.25 ml; 2.0 mmol; 1.1 eq.) est ajouté sur du 3-iodo-1H-pyrazolo[3,4-*d*]pyrimidine-4,6-diamine (500 mg; 1.8 mmol; 1.0 eq.) et de triphénylphosphine (1.43 g; 5.4 mmol; 3.0 eq.) en solution dans du tétrahydrofurane (10 ml). Le milieu réactionnel est refroidi à 0°C puis du diisopropylazodicarboxylate (1.1 ml; 5.4 mmol; 3.0 éq.) est ajouté. Le milieu réactionnel est agité à température ambiante pendant 30 minutes. La réaction est arrêtée par addition d'eau et d'une solution de soude 1N à froid jusqu'à pH basique puis est extraite à l'acétate d'éthyle. La phase organique est lavée avec une solution saturée de chlorure de sodium, séchée sur sulfate de sodium et concentrée. Le produit brut est chromatographié sur gel de silice (25g, dépôt solide, éluant dichlorométhane/méthanol de 0 à 15% de méthanol). Le 1-((S)-1,3-dimethyl-butyl)-3-iodo-1H-pyrazolo[3,4-*d*]pyrimidine-4,6-diamine (308 mg; 47%) est obtenu sous la forme d'une meringue jaune pâle.

### b) 3-(2-amino-6-fluoro-benzooxazol-5-yl)-1-((S)-1,3-dimethyl-butyl)-1H-pyrazolo[3,4-d]pyrimidine-4,6-diamine

Une solution de 1-((S)-1 ,3-dimethyl-butyl)-3-iodo-1H-pyrazolo[3,4-d]pyrimidine-4,6-diamine (230 mg; 0.6 mmol; 1.0 eq.), de 6-fluoro-5-(4,4,5,5-tetramethyl-[1,3,2]dioxaborolan-2-yl)-benzooxazol-2-ylamine (213 mg; 0.8 mmol; 1.2 eq.) et une solution de carbonate de potassium (0.96 ml; 2.0 M; 1.9 mmol; 3.0 eq.) dans du 1,4-dioxane (2.3 ml) est dégazée sous azote pendant 10 minutes puis le 1,1'-bis(diphenylphosphino)ferrocenpalladium(II) dichloride, dichlorométhane (26 mg; 0.03 mmol; 0.05 éq.) est rajouté. Le milieu est chauffé à 110°C pendant 2 heures. La réaction est arrêtée par addition d'eau puis extrait à l'acétate d'éthyle. Les phases organiques sont rassemblées puis lavées avec une solution saturée de chlorure de sodium, séchées sur sulfate de sodium et évaporées. Le produit brut est purifié par HPLC préparative (colonne C18, éluant : acétonitrile dans eau/0.2% de carbonate d'ammonium).

Le 3-(2-amino-6-fluoro-benzooxazol-5-yl)-1-((S)-1,3-dimethyl-butyl)-1*H-*pyrazolo[3,4-*d*]pyrimidine-4,6-diamine (175 mg; 69%) est obtenu sous la forme d'un solide cristallin blanchâtre.

1H RMN (DMSO-d6) δ: 0.80 (d, J = 6.7 Hz, 3H), 0.88 (d, J = 6.5 Hz, 3H), 1.22 - 1.32 (m, 1H), 1.37 (d, J = 6.6 Hz, 3H), 1.52 (ddd, J = 13.4, 8.4, 5.0 Hz, 1H), 1.98 (ddd, J = 13.6, 10.0, 5.4 Hz, 1H), 4.76 (ddd, J = 9.9, 6.9, 5.3 Hz, 1H), 6.10 (s, 4H), 7.17 (d, J = 6.7 Hz, 1H), 7.46 (d, J = 9.2 Hz, 1H), 7.52 (s, 2H)

MS (ESI) m/z = 385 [M+H] ⁺

### Exemple 3: 3-(2-amino-4-fluoro-benzooxazol-5-yl)-1-(1-methyl-cyclobutylmethyl)-1H-pyrazolo[3,4-d]pyrimidine-4,6-diamine

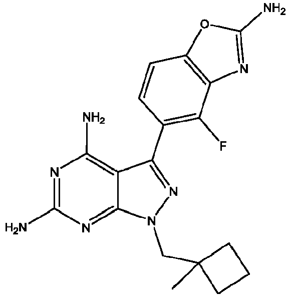

### a) Acide Toluene-4-sulfonique thietan-3-ylmethyl ester

Le chlorure p-toluenesulfonyl (782 mg; 4.1 mmol; 1.5 eq.) est ajouté lentement à 0°C sur le thietan-3-yl-methanol (300 mg; 2.7 mmol; 1.0 eq.), la triéthylamine (1.14 ml; 8.2 mmol; 3.0 eq.) et la 4-diméthylaminopyridine (33 mg; 0.3 mmol; 0.1 eq.) en solution dans du dichlorométhane (5.7 ml). Le milieu réactionnel est agité à température ambiante pendant 1 heure. Le produit brut est chromatographié sur gel de silice (25g, dépôt liquide, éluant heptane/acétate d'éthyle de 0 à 50% d'acétate d'éthyle). L'acide toluene-4-sulfonique thietan-3-ylmethyl ester (631 mg; 89%) est obtenu sous la forme d'une huile incolore.

### b) 3-iodo-1-thietan-3-ylmethyl-1H-pyrazolo[3,4-d]pyrimidine-4,6-diamine

L'acide toluene-4-sulfonique thietan-3-ylmethyl ester (618 mg; 2.4 mmol; 1.1 eq.) est ajouté sur une suspension de 3-iodo-1H-pyrazolo[3,4-*d*]pyrimidine-4,6-diamine (600 mg; 2.2 mmol; 1.0 éq.) et de carbonate de césium (1.42 g; 4.4 mmol; 2.0 eq.) dans du *N*,*N*'-diméthylformamide (6.0 ml). Le milieu réactionnel est chauffé à 120°C aux micro-ondes pendant 20 minutes. La réaction est arrêtée par addition d'eau puis extraite à l'acétate d'éthyle. Les phases organiques sont rassemblées, lavées avec une solution saturée de chlorure de sodium, séchées sur sulfate de sodium et évaporées. Le résidu est repris dans de l'éthanol et est filtré. Le 3-iodo-1-thietan-3-ylmethyl-1H-pyrazolo[3,4-*d*]pyrimidine-4,6-diamine (412 mg; 52%) est obtenu sous la forme d'une poudre orange.

### c) 3-(2-amino-4-fluoro-benzooxazol-5-yl)-1-(1-methyl-cyclobutylmethyl)-1H-pyrazolo[3,4-d]pyrimidine-4,6-diamine

Une solution de 3-iodo-1-(1-methyl-cyclobutylmethyl)-1H-pyrazolo[3,4-d]pyrimidine-4,6-diamine (200 mg; 0.6 mmol; 1.0 eq.), de 4-fluoro-5-(4,4,5,5-tetramethyl-[1,3,2]dioxaborolan-2-yl)-benzooxazol-2-ylamine (186 mg; 0.7 mmol; 1.2 eq.) et une solution de carbonate de potassium (0.84 ml; 2.0 M; 1.7 mmol; 3.0 eq.) dans du 1,4-dioxane (2.0 ml) est dégazée sous azote pendant 10 minutes puis le 1,1'-bis(diphenylphosphino)ferrocenepalladium(II) dichloride, dichlorométhane (23 mg; 0.03 mmol; 0.05 eq.) est rajouté. Le milieu est chauffé à 110°C pendant 30 minutes. La réaction est arrêtée par addition d'eau puis extraite à l'acétate d'éthyle. Les phases organiques sont rassemblées, lavées avec une solution saturée de chlorure de sodium, séchées sur sulfate de sodium et évaporés. Le produit brut est chromatographié sur gel de silice (25g, dépôt solide, éluant dichlorométhane/méthanol de 0 à 15% de méthanol). Le produit, n'étant pas pur, est repurifié par HPLC préparative (colonne C18, éluant : acétonitrile dans eau/0.1% d'acide formique). Le 3-(2-amino-4-fluoro-benzooxazol-5-yl)-1-(1-methyl-cyclobutylmethyl)-1H-pyrazolo[3,4-*d*]pyrimidine-4,6-diamine (81 mg; 38%) est obtenu sous la forme d'un solide blanc.

1H RMN (DMSO-d6) δ: 1.11 (s, 3H), 1.58 - 1.74 (m, 3H), 1.84 (tdd, J = 7.5, 5.9, 2.1 Hz, 1H), 2.16 (qd, J = 8.8, 8.0, 3.9 Hz, 2H), 4.09 (s, 2H), 6.13 (s, 2H), 7.04 (dd, J = 8.2, 6.6 Hz, 1H), 7.30 (d, J = 8.1 Hz, 1H), 7.68 (s, 2H)

MS (ESI) m/z = 383 [M+H] ⁺

### Exemple 4: 3-(2-amino-6-fluoro-benzooxazol-5-yl)-1-(1-methyl-cyclobutylmethyl)-1H-pyrazolo[3,4-d]pyrimidine-4,6-diamine

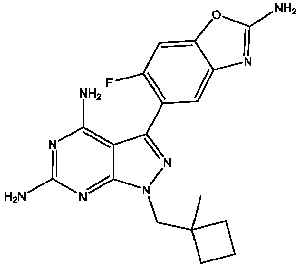

1H RMN (DMSO-d6) δ: 1.10 (s, 3H), 1.59 - 1.69 (m, 3H), 1.80 - 1.88 (m, 1H), 4.08 (s, 2H), 6.13 (s, 4H), 7.16 - 7.18 (d, J = 6.6 Hz, 1H), 7.46 - 7.48 (d, J = 9.2 Hz, 1H), 7.52 (s, 2H).

MS (ESI) m/z = 383 [M+H] ⁺

Les composés des exemples 4-18, 20, 21, 22, 25 et 28-30 peuvent être obtenus selon le procédé décrit dans la Figure 1 en utilisant des précurseurs sur lesquels les groupements R₁, R₂, R₄ ou R₅ ont été préalablement introduits (*cf*. Figure 1).

### Exemple 5 : 3-(2-amino-4-fluoro-benzooxazol-5-yl)-1-((S)-1,3-dimethyl-butyl)-1H-pyrazolo[3,4-d]pyrimidine-4,6-diamine

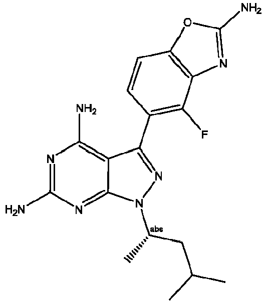

### a) 1-((S)-1,3-dimethyl-butyl)-3-iodo-1H-pyrazolo[3,4-d]pyrimidine-4,6-diamine

Du (R)-4-methyl-pentan-2-ol (0.25 ml; 2.0 mmol; 1.1 eq.) est ajouté sur du 3-iodo-1H-pyrazolo[3,4-*d*]pyrimidine-4,6-diamine (500 mg; 1.8 mmol; 1.0 eq.) et de triphénylphosphine (1.43 g; 5.4 mmol; 3.0 eq.) en solution dans du tétrahydrofurane (10 ml). Le milieu réactionnel est refroidi à 0°C puis du diisopropylazodicarboxylate (1.1 ml; 5.4 mmol; 3.0 eq.) est ajouté. Le milieu réactionnel est agité à température ambiante pendant 30 minutes. La réaction est arrêtée par addition d'eau et d'une solution de soude 1N à froid jusqu'à pH basique puis est extraite à l'acétate d'éthyle. La phase organique est lavée avec une solution saturée de chlorure de sodium, séchée sur sulfate de sodium et concentrée. Le produit brut est chromatographié sur gel de silice (25g, dépôt solide, éluant dichlorométhane/méthanol de 0 à 15% de méthanol). Le 1-((S)-1,3-dimethyl-butyl)-3-iodo-1H-pyrazolo[3,4-*d*]pyrimidine-4,6-diamine (308 mg; 47%) est obtenu sous la forme d'une meringue jaune pâle.

### b) 3-(2-Amino-4-fluoro-benzooxazol-5-yl)-1-((S)-1,3-dimethyl-butyl)-1H-pyrazolo[3,4-d]pyrimidine-4,6-diamine

Une solution de 1-((S)-1 ,3-dimethyl-butyl)-3-iodo-1H-pyrazolo[3,4-d]pyrimidine-4,6-diamine (160 mg; 0.4 mmol; 1.0 eq.), de 4-fluoro-5-(4,4,5,5-tetramethyl-[1,3,2]dioxaborolan-2-yl)-benzooxazol-2-ylamine (148 mg; 0.5 mmol; 1.2 eq.) et une solution de carbonate de potassium (0.67 ml; 2.0 M; 1.3 mmol; 3.0 eq.) dans du 1,4-dioxane (1.6 ml) est dégazée sous azote pendant 10 minutes puis le 1,1'-bis(diphenylphosphino)ferrocenepalladium(II) dichloride, dichlorométhane (18 mg; 0.02 mmol; 0.05 eq.) est rajouté. Le milieu est chauffé à 110°C pendant 30 minutes. La réaction est arrêtée par addition d'eau puis extrait à l'acétate d'éthyle. Les phases organiques sont rassemblées, lavées avec une solution saturée de chlorure de sodium, séchées sur sulfate de sodium et évaporées. Le produit brut est chromatographié sur gel de silice (25g, dépôt solide, éluant dichlorométhane/méthanol de 0 à 15% de méthanol). Le 3-(2-amino-4-fluoro-benzooxazol-5-yl)-1-((S)-1,3-dimethyl-butyl)-1H-pyrazolo[3,4-*d*]pyrimidine-4,6-diamine (75 mg; 44%) est obtenu sous la forme d'un solide cristallin beige après recristallisation dans acétonitrile/eau. 1H RMN (DMSO-d6) δ: 0.80 (d, J = 6.5 Hz, 3H), 0.88 (d, J = 6.5 Hz, 3H), 1.28 (dt, J = 14.4, 6.8 Hz, 1H), 1.37 (d, J = 6.7 Hz, 3H), 1.52 (ddd, J = 13.4, 8.5, 5.0 Hz, 1H), 1.98 (ddd, J = 14.2, 9.9, 5.3 Hz, 1H), 4.77 (dt, J = 10.8, 5.9 Hz, 1H), 6.10 (s, 4H), 6.98 - 7.11 (m, 1H), 7.30 (d, J = 8.3 Hz, 1H), 7.67 (s, 2H)

MS (ESI) m/z = 325 [M+H] ⁺

### Exemple 6 : N-(5-(4,6-diamino-1-(2,2-dimethylbutyl)-1H-pyrazolo[3,4-d]pyrimidin-3-yl)benzo[d]oxazol-2-yl)acetamide

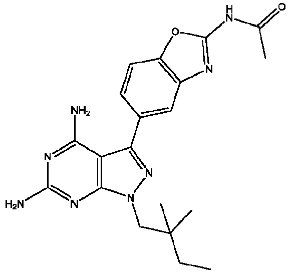

1H RMN (DMSO-d6) δ: 0.89 (t, J=7.5 Hz, 3 H), 0.91 (s, 6 H), 1.33 (q, J=7.5 Hz, 2 H), 2.23 (s, 3 H), 3.95 (s, 2 H), 6.12-6.36 (br s, 4 H), 7.48 (dd, J=8.4, 1.8 Hz, 1 H), 7.68 - 7.74 (m, 2 H), 11.70 (br s, 1 H)

MS (ESI) m/z = 409 [M+H] ⁺

### Exemple 7: 3-(2-amino-benzooxazol-5-yl)-1-(2,2-dimethyl-propyl)-1H-pyrazolo[3,4-d]pyrimidine-4,6-diamine

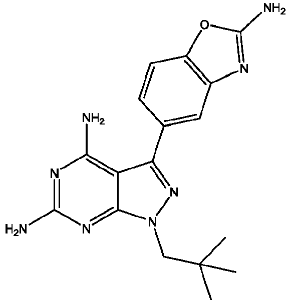

### a) 1-(2,2-dimethyl-propyl)-3-iodo-1H-pyrazolo[3,4-d]pyrimidine-4,6-diamine

Du carbonate de césium (2.36 g; 7.3 mmol; 2.0 eq.) puis du 1-bromo-2,2-dimethylpropane (502 µl; 4.0 mmol; 1.1 eq.) sont ajoutés à une solution de 3-iodo-1H-pyrazolo[3,4-*d*]pyrimidine-4,6-diamine (1.00 g; 3.6 mmol; 1.0 eq.) dans *N*,*N*'-dimethylformamide (20 ml). Le milieu réactionnel est chauffé à 110°C pendant 2 jours. Le milieu réactionnel refroidi est hydrolysé avec de l'eau et extrait à l'acétate d'éthyle. Les phases organiques sont rassemblées, lavées à l'eau, séchées sur sulfate de magnésium, filtrées et évaporées. Le résidu est chromatographié sur gel de silice élué au dichlorométhane / méthanol (98/2 à 95/5). Le 1-(2,2-dimethyl-propyl)-3-iodo-1H-pyrazolo[3,4-*d*]pyrimidine-4,6-diamine (1.04 g; 83%) est obtenu sous forme d'un solide blanc.

### b) 3-(2-amino-benzooxazol-5-yl)-1-(2,2-dimethyl-propyl)-1H-pyrazolo[3,4-d]pyrimidine-4,6-diamine

Une solution de 1-(2,2-dimethyl-propyl)-3-iodo-1H-pyrazolo[3,4-*d*]pyrimidine-4,6-diamine (323 mg; 0.9 mmol; 1.0 eq.), de 5-(4,4,5,5-tetramethyl-1,3,2-dioxaborolan-2-yl)benzo[d]oxazol-2-amine (364 mg; 1.4 mmol; 1.5 eq.) et une solution de carbonate de potassium (1.4 ml; 2.0 M; 2.8 mmol; 3.0 eq.) dans du 1,4-dioxane (3.2 ml) est dégazée sous azote pendant 10 minutes puis le 1,1'-bis(diphenylphosphino)ferrocenepalladium(II) dichloride, dichlorométhane (152 mg; 0.19 mmol; 0.20 eq.) est rajouté. Le milieu est chauffé à 110°C pendant 30 minutes. La phase aqueuse est éliminée et le milieu est filtré sur célite. Le solvant est évaporé et le résidu est chromatographié sur gel de silice élué au dichlorométhane / méthanol (95/5 à 80/20). Le solvant est concentré et l'insoluble est filtré et séché sous vide à 40°C. Le 3-(2-amino-benzooxazol-5-yl)-1-(2,2-dimethyl-propyl)-1H-pyrazolo[3,4-*d*]pyrimidine-4,6-diamine (160 mg; 48%) est obtenu sous forme d'un solide beige saumon.

1H RMN (DMSO-d6) δ: 0.97 (s, 9H), 3.93 (s, 2H), 6.12 (s, 4H), 7.19-7.21 (dd, J = 8.1, 1.7 Hz, 1H), 7.37 (d, J = 1.5 Hz, 1H), 7.42-7.44 (d, J = 8.1 Hz, 1H), 7.49 (s, 2H).

MS (ESI) m/z = 353 [M+H] ⁺

### Exemple 8: 3-(2-amino-benzooxazol-5-yl)-1-(2,2-dimethyl-butyl)-1H-pyrazolo[3,4-d]pyrimidine-4,6-diamine

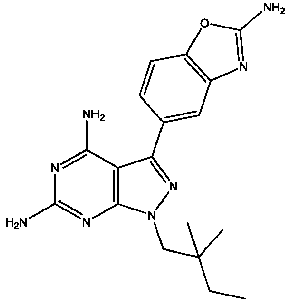

### a) 1-(2,2-dimethyl-butyl)-3-iodo-1H-pyrazolo[3,4-d]pyrimidine-4,6-diamine

Du di-*ter*t-butyl azodicarboxylate (1.25 g; 5.4 mmol; 1.5 eq.) est ajouté à une solution refroidie à 0°C de triphénylphosphine (1.43 g; 5.4 mmol; 1.5 eq.) dans du toluène (10 ml). Le milieu réactionnel est agité à 0°C pendant 30 minutes. Du 2,2-diméthyl-butan-1-ol (555 mg; 5.4 mmol; 1.5 eq.) est ensuite ajouté goutte à goutte et le milieu réactionnel est agité à 0°C pendant 30 minutes. Enfin, du 3-iodo-1H-pyrazolo[3,4-d]pyrimidine-4,6-diamine (1.00 g; 3.6 mmol; 1.0 éq.) est ajouté et le milieu réactionnel est chauffé à 110°C pendant 1 heure 15 minutes.

Cette étape est refaite mais avec du di-tert-butyl azodicarboxylate (0.42 g; 1.8 mmol; 0.5 eq.), de la triphénylphosphine (0.48 g; 1.8 mmol; 0.5 eq.) et du 2,2-diméthyl-butan-1-ol (185 mg; 1.8 mmol; 0.5 eq.) dans 3.5 ml de toluène. Cette solution est ajoutée sur le milieu réactionnel qui est chauffé à 110°C pendant 1h10.

Le milieu réactionnel est concentré sous vide. Le résidu est repris avec une solution d'acide acétique (8 ml) et d'eau (2 ml). Le milieu réactionnel est chauffé à 100°C pendant 1h30. Le milieu réactionnel est évaporé sous azote et le résidu est chromatographié sur gel de silice élué au dichlorométhane / méthanol (98/2 à 95/5). Le 1-(2,2-diméthyl-butyl)-3-iodo-1*H*-pyrazolo[3,4-*d*]pyrimidine-4,6-diamine (0.81 g; 62%) est obtenu sous forme d'un solide jaune.

### b) 3-(2-amino-benzooxazol-5-yl)-1-(2,2-dimethyl-butyl)-1H-pyrazolo[3,4-d]pyrimidine-4,6-diamine

Une solution de 1-(2,2-dimethyl-butyl)-3-iodo-1*H*-pyrazolo[3,4-*d*]pyrimidine-4,6-diamine (880 mg; 2.4 mmol; 1.0 eq.), de 5-(4,4,5,5-tetramethyl-1,3,2-dioxaborolan-2-yl)benzo[d]oxazol-2-amine (953 mg; 3.7 mmol; 1.5 eq.) et une solution de carbonate de potassium (3.7 ml; 2.0 M; 7.3 mmol; 3.0 eq.) dans du 1,4-dioxane (8.8 ml) est dégazée sous azote pendant 10 minutes puis le 1,1'-bis(diphenylphosphino)ferrocenepalladium(II) dichloride, dichlorométhane (399 mg; 0.5 mmol; 0.20 eq.) est rajouté. Le milieu réactionnel est chauffé à 1 10°C pendant 45 minutes. La phase aqueuse est éliminée et le milieu est filtré sur célite. Le résidu est purifié par HPLC préparative en condition acide (25 à 35% ACN). L'acétonitrile est évaporé et la phase aqueuse est neutralisée avec une solution saturée d'hydrogénocarbonate de sodium et extraite à l'acétate d'éthyle. Les phases organiques sont rassemblées, lavées à l'eau, séchées sur sulfate de magnésium, filtrées et concentrées. Le solide est filtré et séché sous vide à 40°C. Le 3-(2-amino-benzooxazol-5-yl)-1-(2,2-dimethyl-butyl)-1H-pyrazolo[3,4-d]pyrimidine-4,6-diamine (414 mg; 46%) est obtenu sous forme d'un solide beige pâle.

1H RMN (DMSO-d6) δ: 0.87-0.91 (t, J = 7.6 Hz, 3H), 0.91 (s, 6H), 1.30-1.35 (q, J = 7.6 Hz, 2H), 3.94 (s, 2H), 6.10 (s, 4H), 7.19-7.21 (dd, J = 8.1, 1.7 Hz, 1H), 7.36 (d, J = 1.6 Hz, 1H), 7.42-7.44 (d, J = 8.1 Hz, 1H), 7.49 (s, 2H).

MS (ESI) m/z = 367 [M+H] ⁺

### Exemple 9: 3-(2-amino-benzooxazol-5-yl)-1-(3-methyl-thietan-3-ylmethyl)-1H-pyrazolo[3,4-d]pyrimidine-4,6-diamine

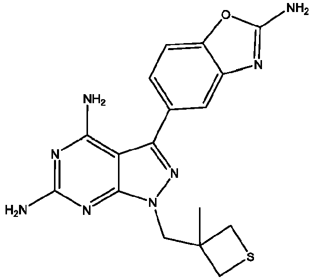

1H RMN (DMSO-d6) δ: 1.29 (s, 3H), 2.79-2.81 (d, J = 9.2 Hz, 2H), 3.43-3.46 (d, J = 9.4 Hz, 2H), 4.22 (s, 2H), 6.19 (s, 4H), 7.20-7.22 (d, J = 8.0 Hz, 1H), 7.37 (s, 1H), 7.43-7.45 (d, J = 8.1 Hz, 1H), 7.50 (s, 2H).

MS (ESI) m/z = 383 [M+H] ⁺

### Exemple 10 : 1-(2,2-dimethyl-butyl)-3-iodo-1H-pyrazolo[3,4-d] pyrimidine-4,6-diamine

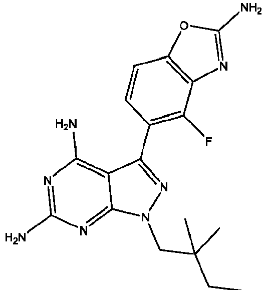

1H RMN (DMSO-d6) δ: 0.89 (m, 9H), 1.32 (q, J = 7.4 Hz, 2H), 3.95 (s, 2H), 6.10 (s, 3H), 7.04 (dd, J = 8.2, 6.6 Hz, 1H), 7.30 (d, J = 8.2 Hz, 1H), 7.68 (s, 2H).

MS (ESI) m/z = 385 [M+H] ⁺

### Exemple 11 : 1-(2,2-dimethyl-propyl)-3-(2-ethylamino-benzooxazol-5-yl)-1H-pyrazolo[3,4-d]pyrimidine-4,6-diamine

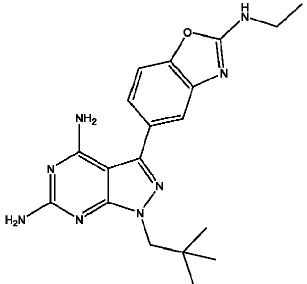

1H RMN (DMSO-d6) δ: 0.97 (s, 9 H), 1.20 (t, J=7.2 Hz, 3 H), 3.36 (qd, J=7.2, 5.7 Hz, 2 H), 3.93 (s, 2 H), 6.11 (br s, 4 H), 7.20 (dd, J=8.1, 1.8 Hz, 1 H), 7.39 (dd, J=1.8, 0.4 Hz, 1 H), 7.44 (dd, J=8.1, 0.4 Hz, 1 H), 8.00 (t, J=5.7 Hz, 1 H).

MS (ESI) m/z = 381 [M+H] ⁺

### Exemple 12 : 3-(2-amino-benzooxazol-5-yl)-1-(2,2-dimethyl-but-3-enyl)-1H-pyrazolo[3,4-d]pyrimidine-4,6-diamine

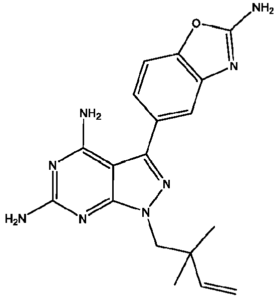

1H RMN (DMSO-d6) δ: 1.08 (s, 6H), 4.03 (s, 2H), 4.86 - 5.09 (m, 2H), 6.00 (dd, J = 17.5, 10.8 Hz, 1H), 6.13 (s, 3H), 7.20 (dd, J = 8.1, 1.7 Hz, 1H), 7.36 (d, J = 1.7 Hz, 1H), 7.44 (d, J = 8.2 Hz, 1H), 7.50 (s, 2H)

MS (ESI) m/z = 365 [M+H] ⁺

### Exemple 13: (+)-3-(2-amino-benzooxazol-5-yl)-1-((S)-2-methyl-2-tetrahydro-furan-2-yl-propyl)-1H-pyrazolo[3,4-d]pyrimidine-4,6-diamine

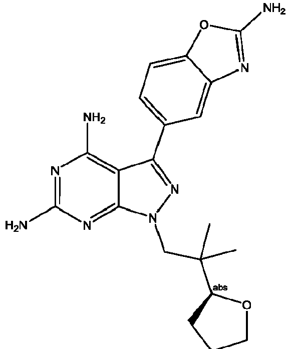

1H RMN (DMSO-d6) δ: 0.88 - 0.89 (d, J = 1.7 Hz, 6H), 1.63-1.70 (m, 1H), 1.78-1.91 (m, 3H), 3.63-3.72 (m, 2H), 3.74-3.79 (m, 1H), 3.97-4.00 (d, J = 13.8 Hz, 1H), 4.12-4.15 (d, J = 13.8 Hz, 1H), 6.15 (s, 4H), 7.19-7.21 (dd, J = 8.1, 1.7 Hz, 1H), 7.36-7.37 (d, J = 1.2 Hz, 1H), 7.43-7.45 (d, J = 8.1 Hz, 1H), 7.52 (s, 2H).

MS (ESI) m/z = 409 [M+H] ⁺

### Exemple 14 : 3-(2-amino-benzooxazol-5-yl)-1-(2,2-dimethyl-but-3-ynyl)-1H-pyrazolo[3,4-d]pyrimidine-4,6-diamine

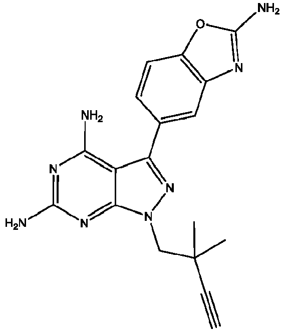

1H RMN (DMSO-d6) δ: 1.26 (s, 6H), 2.99 (s, 1H), 4.18 (s, 2H), 5.96 - 6.47 (m, 4H), 7.21 (dd, J = 8.1, 1.7 Hz, 1H), 7.37 (d, J = 1.7 Hz, 1H), 7.44 (d, J = 8.1 Hz, 1H), 7.51 (s, 2H)

MS (ESI) m/z = 363 [M+H] ⁺

### Exemple 15 : 3-[4,6-diamino-3-(2-amino-benzooxazol-5-yl)-pyrazolo[3,4-d]pyrimidin-1-yl]-2,2-dimethyl-propan-1-ol

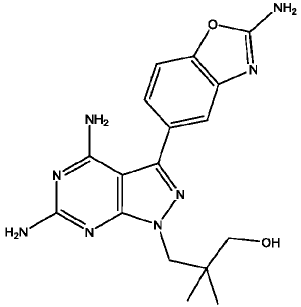

1H RMN (DMSO-d6) δ: 0.89 (s, 6H), 3.10 (d, J = 6.2 Hz, 2H), 5.08 (t, J = 6.6 Hz, 1H), 6.27 (s, 4H), 7.20 (dd, J = 8.0, 1.8 Hz, 1H), 7.36 (d, J = 1.8 Hz, 1H), 7.44 (d, J = 8.0 Hz, 1H), 7.50 (s, 2H)

MS (ESI) m/z = 369 [M+H] +

### Exemple 16: 3-(2-amino-benzooxazol-5-yl)-1-(2-methyl-2-methylsulfanyl-propyl)-1H-pyrazolo[3,4-d]pyrimidine-4,6-diamine

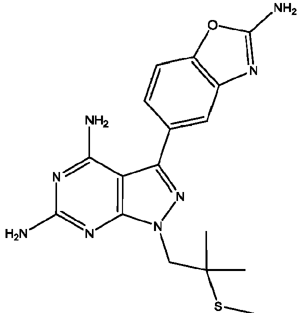

1H RMN (DMSO-d6) δ: 1.74 (s, 6H), 1.85 (s, 3H), 3.35 (s, 2H), 6.06 (s, 4H), 7.16-7.18 (dd, J = 8.1, 1.4 Hz, 1H), 7.34 (d, J = 1.2 Hz, 1H), 7.42-7.44 (d, J = 8.1 Hz, 1H), 7.49 (s, 2H).

MS (ESI) m/z = 385 [M+H] ⁺

### Exemple 17: 3-(2-amino-4-chloro-benzooxazol-5-yl)-1-(2,2-dimethyl-propyl)-1H-pyrazolo[3,4-d]pyrimidine-4,6-diamine

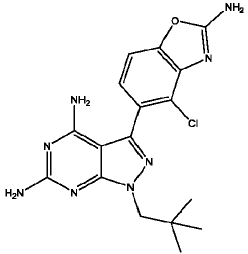

1H RMN (DMSO-d6) δ: 0.97 (s, 9H), 3.94 (s, 2H), 6.10 (s, 4H), 7.02 - 7.04 (d, J = 7.9 Hz, 1H), 7.39 - 7.41 (d, J = 8.1 Hz, 1H), 7.77 (s, 2H).

MS (ESI) m/z = 387 [M+H] +

### Exemple 18 : 3-(2-amino-6-methoxy-benzooxazol-5-yl)-1-(2,2-dimethyl-propyl)-1H-pyrazolo[3,4-d]pyrimidine-4,6-diamine

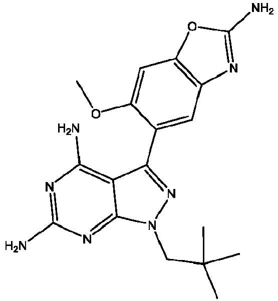

1H RMN (DMSO-d6) δ: 0.97 (s, 9H), 3.76 (s, 3H), 3.91 (s, 2H); 6.03 (s, 2H), 7.09 (s, 1H), 7.29 (d, J = 3.0 Hz, 3H)

MS (ESI) m/z = 383 [M+H] ⁺

### Exemple 19: 2-[4,6-diamino-1-(2,2-dimethyl-propyl)-1H-pyrazolo[3,4-d]pyrimidin-3-yl]-1H-pyrrolo[2,3-b]pyridin-5-ol

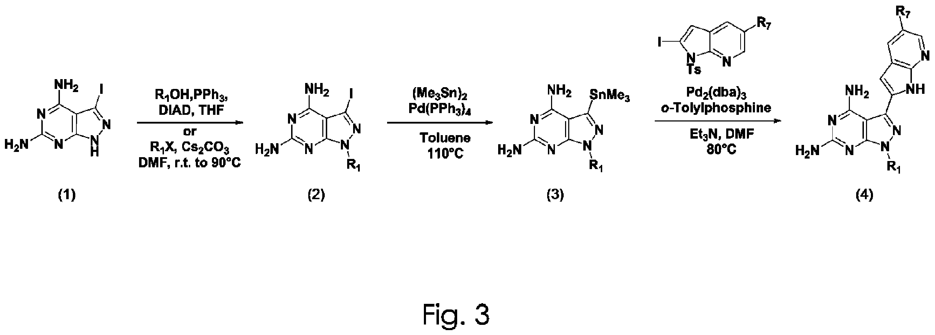

Ce composé peut être obtenu selon le procédé présenté dans la Figure 3.

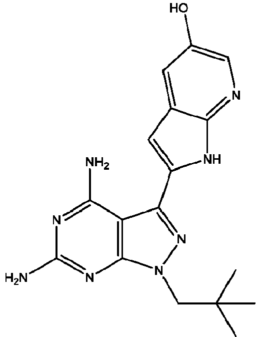

### a) 1-Neopentyl-3-(trimethylstannyl)-1H-pyrazolo[3,4-d]pyrimidine-4,6-diamine

A une solution de 1-(2,2-dimethylpropyl)-3-iodo-pyrazolo[3,4-d]pyrimidine-4,6-diamine (1.37 g; 3.97 mmol; 1 eq.) dans le toluène (0.15 M) dégazé à l'argon, est ajouté de l'hexaméthyldistannane (1.56 g, 4.75 mmol; 1.2 eq.) suivi de tétrakis(triphénylphosphine) palladium (0) (0.2 mmol ; 0.05 eq.). Le mélange est agité à 110°C pendant 30 min. Le mélange est directement concentré sous vide et le résidu purifié sur gel d'alumine basique pour fournir le 1-neopentyl-3-(trimethylstannyl)-1*H*-pyrazolo[3,4-*d*]pyrimidine-4,6-diamine (792 mg; 2.06 mmol; 52%) sous la forme d'un solide marron.

### b) 3-(5-Methoxy-1-(phenylsulfonyl)-1H-pyrrolo[2,3-b]pyridin-2-yl)-1-neopentyl-1H-pyrazolo[3,4-d]pyrimidine-4,6-diamine

A une solution de 3-trimethylstannyl-pyrazolo[3,4-*d*]pyrimidine-4,6-diamine (1 eq.) dans le *N*,*N*'-diméthylformamide (0.14 M) dégazé à l'argon est ajouté du 2-iodo-5-methoxy-1-(phenylsulfonyl)-1*H*-pyrrolo[2,3-*b*]pyridine (1.6 mmol, 1 eq.) suivi du tris(dibenzylideneacetone)dipalladium (0) (0.16 mmol; 0.1 eq), du tri-o-tolylphosphine (0.48 mmol; 0.3 eq.) et de la triethylamine (0.48 mmol; 3 eq.). Le mélange est ensuite agité à 80°C pendant 2h pour obtenir une conversion totale. Après retour à température ambiante, le mélange est dilué par addition d'eau puis la phase aqueuse est extraite à l'acétate d'éthyle. Les phases organiques sont rassemblées, lavées avec une solution saturée de chlorure de sodium, séchées sur sulfate de sodium et concentrées. Le produit brut est chromatographié sur gel de silice. Le 3-(5-Methoxy-1-(phenylsulfonyl)-1*H*-pyrrolo[2,3-*b*]pyridin-2-yl)-1-neopentyl-1*H*-pyrazolo[3,4-*d*]pyrimidine-4,6-diamine (234 mg; 29%) est obtenu sous la forme d'un solide incolore.

### c) 2-[4,6-Diamino-1-(2,2-dimethyl-propyl)-1H-pyrazolo[3,4-d]pyrimidin-3-yl]-1H-pyrrolo[2,3-b]pyridin-5-ol

A une solution de 3-(5-Methoxy-1-(phenylsulfonyl)-1*H*-pyrrolo[2,3-*b*]pyridin-2-yl)-1-neopentyl-1*H*-pyrazolo[3,4-d]pyrimidine-4,6-diamine (1 eq.) dans le méthanol (0.5 M) est ajouté du carbonate de potassium (2 eq.). Le mélange est agité à 65°c jusqu'à totale conversion et dilué avec un mélange 1 :1 eau : acétate d'éthyle. La phase aqueuse est extraite à l'acétate d'éthyle, les phases organiques sont rassemblées, lavées avec une solution saturée de chlorure de sodium, séchées sur sulfate de sodium et concentrées. Le produit est directement engagé dans l'étape suivante sans plus de purification.

A une solution de 3-(5-methoxy-1*H*-pyrrolo[2,3-*b*]pyridin-2-yl)-1-neopentyl-1*H-*pyrazolo[3,4-*d*]pyrimidine-4,6-diamine (1 eq.) dans le dichlorométhane (0.06 M) à -78°C est ajouté une solution de tribromure de bore (1 M dans le dichlorométhane; 9 eq.). Le mélange réactionnel est agité pendant 14h avec retour progressif à température ambiante et la réaction est arrêtée par addition d'une solution saturée de bicarbonate de sodium à 0°C puis la phase aqueuse est extraite à l'acétate d'éthyle. Les phases organiques sont rassemblées, lavées avec une solution saturée de chlorure de sodium, séchées sur sulfate de sodium et concentrées. Le produit brut est chromatographié sur gel de silice. Le 2-[4,6-Diamino-1-(2,2-dimethyl-propyl)-1H-pyrazolo[3,4-d]pyrimidin-3-yl]-1H-pyrrolo[2,3-b]pyridin-5-ol (16.5 mg; 44%) est obtenu sous la forme d'un solide incolore.

1H RMN (DMSO-d6) δ: 0.98 (s, 9H), 3.96 (s, 2H), 6.18 (s, 2H), 6.45 (s, 2H), 6.58 (s, 1H), 7.33 (d, J = 2.61 Hz, 1H), 7.88 (d, J = 2.61 Hz, 1H), 9.15 (s, 1H), 11.70 (s, 1H).

MS (ESI) m/z = 353 [M+H]⁺

### Exemple 20 : 3-(2-amino-6-methoxy-benzooxazol-5-yl)-1-(2,2-dimethyl-butyl)-1H-pyrazolo [3,4-d] pyrimidine-4,6-dia mine

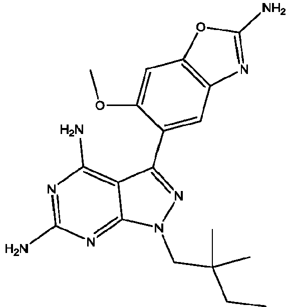

1H RMN (DMSO-d6) δ: 0.90 (s, 9H), 1.31-1.33 (d, J = 6.2 Hz, 2H), 3.75 (s, 3H), 3.91 (s, 2H), 6.03 (s, 4H), 7.07 (s, 1H), 7.28 (s, 1H), 7.30 (s, 2H).

MS (ESI) m/z = 397 [M+H] ⁺

### Exemple 21 : 3-(2-amino-benzooxazol-5-yl)-1-isopropyl-1H-pyrazolo[3,4-d] pyrimidine-4,6-diamine

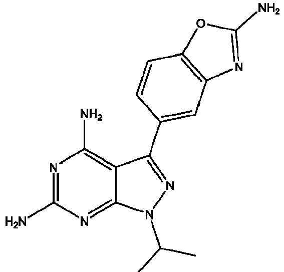

1H RMN (DMSO-d6) δ: 1.43 (d, J = 6.7 Hz, 6H), 4.79-4.85 (p, J = 6.7 Hz, 1H), 6.14 (s, 2H), 7.18-7.21 (dd, J = 8.1-1.7 Hz, 1H), 7.36 (d, J = 1.7 Hz, 1H), 7.43 (d, J = 8.1 Hz, 1H), 7.50 (s, 2H).

MS (ESI) m/z = 325 [M+H] ⁺

### Exemple 22: 3-(2-amino-benzooxazol-5-yl)-1-(2-ethyl-2-methanesulfonyl-butyl)-1H-pyrazolo[3,4-d]pyrimidine-4,6-diamine

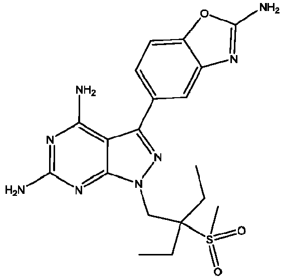

1H RMN (DMSO-d6) δ: 1.02 (t, J = 7.4 Hz, 6H), 1.90 (p, J = 7.3 Hz, 4H), 3.09 (s, 3H), 4.54 (s, 2H), 6.23 (s, 2H), 7.20 (dd, J = 8.1, 1.7 Hz, 1H), 7.37 (d, J = 1.7 Hz, 1H), 7.45 (d, J = 8.1 Hz, 1H), 7.51 (s, 2H)

MS (ESI) m/z = 445 [M+H] ⁺

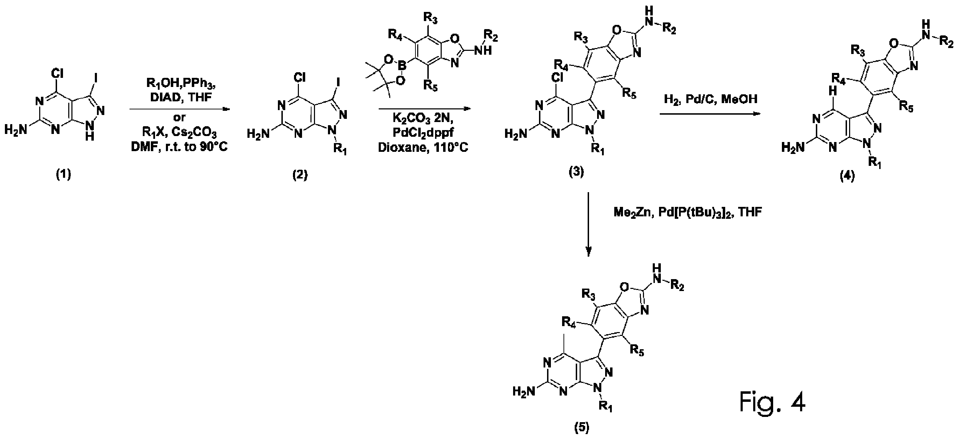

### Exemple 23 : 3-(2-amino-benzooxazol-5-yl)-4-chloro-1-isobutyl-1H-pyrazolo[3,4-d]pyrimidin-6-ylamine Ce composé peut être obtenu selon le procédé présenté dans la Figure 4.

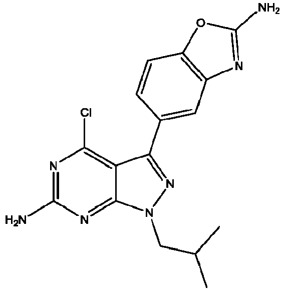

1H RMN (DMSO-d6) δ: 0.89 (d, J=6.8 Hz, 6 H), 2.19 - 2.32 (m, 1 H), 4.03 (d, J=7.3 Hz, 2 H), 7.24 - 7.30 (m, 3 H), 7.40 (d, J=8.2 Hz, 1 H), 7.42 - 7.50 (m, 3 H).

MS (ESI) m/z = 358 [M+H] ⁺

### Exemple 24 : 3-(2-amino-benzooxazol-5-yl)-1-isopropyl-1 H-pyrazolo[3,4-d]pyrimidin-6-ylamine Ce composé peut être obtenu selon le procédé présenté dans la Figure 4.

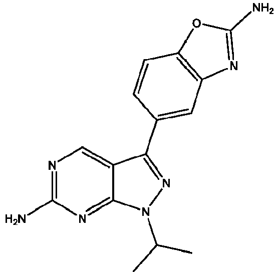

1H RMN (DMSO-d6) δ: 1H NMR (400 MHz, DMSO-d6) δ 1.48 (d, J = 6.7 Hz, 6H), 4.90 (p, J = 6.6 Hz, 1H), 6.90 (s, 2H), 7.41 (d, J = 8.1 Hz, 1H), 7.50 (s, 2H), 7.59 (dd, J = 8.2, 1.7 Hz, 1H), 7.72 (d, J = 1.7 Hz, 1H), 9.06 (s, 1H).

MS (ESI) m/z = 310 [M+H] ⁺

### Exemple 25 : 3-(2-amino-6-chloro-benzooxazol-5-yl)-1-((S)-1,2-dimethyl-propyl)-1H-pyrazolo[3,4-d]pyrimidine-4,6-diamine

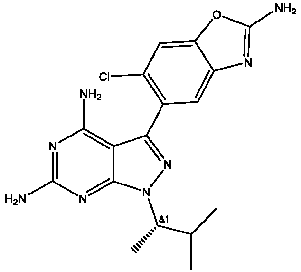

1H RMN (DMSO-d6) δ: 0.69 (d, J = 6.7 Hz, 3H), 0.97 (d, J = 6.7 Hz, 3H), 1.41 (d, J = 6.8 Hz, 3H), 2.13 (dt, J = 8.8, 6.6 Hz, 1H), 4.28 - 4.40 (m, 1H), 6.09 (s, 2H), 7.18 (s, 1H), 7.63 (s, 1H), 7.65 (s, 2H)

MS (ESI) m/z = 387 [M+H] ⁺

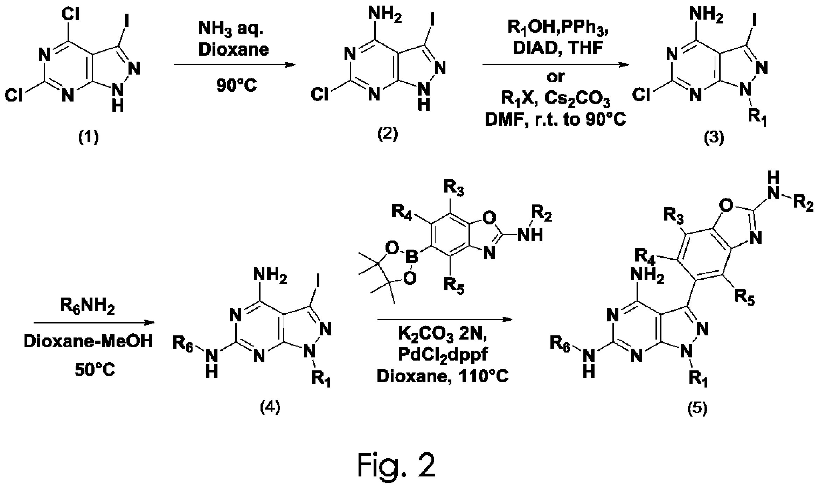

### Exemple 26: 3-(2-Amino-benzooxazol-5-yl)-1-isopropyl-N⁶-methyl-1H-pyrazolo[3,4-d]pyrimidine-4,6-diamine Ce composé peut être obtenu selon le procédé présenté dans la Figure 2.

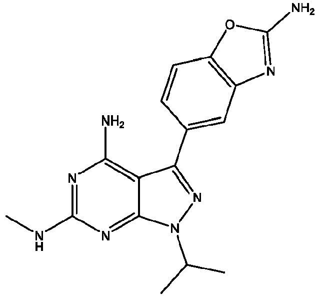

### a) 6-Chloro-3-iodo-1H-pyrazolo[3,4-d]pyrimidin-4-ylamine

4,6-Dichloro-3-iodo-1*H*-pyrazolo[3,4-*d*]pyrimidine (15.8 g; 50.2 mmol; 1 eq.) (1) est placé en solution dans 1,4-dioxane (480 mL; 0.1 M). Une solution d'Ammoniaque aqueux (474.00 ml; 32.00% g/g; 890.61 mmol; 30.00 V) est additionné et le tout est chauffé à 90°C pendant 1h. Après retour à température ambiante, le milieu hétérogène est concentré sous vide et le solide orange obtenu est repris dans l'acétate d'éthyle puis filtré pour fournir le produit désiré (14 g, 93%) sous la forme d'un solide beige.

### b) 6-Chloro-3-iodo-1H-pyrazolo[3,4-d]pyrimidin-4-ylamine

A une solution de 6-Chloro-3-iodo-1*H*-pyrazolo[3,4-*d*]pyrimidin-4-ylamine (500 mg; 1.69 mmol; 1eq.) (2) dans *N,N*'dimethylformamide (7 mL) est ajouté césium carbonate (1.10 g; 3.38 mmol; 2eq.) suivi du 2-iodopropane (0.17 mL; 1.69 mmol; 1eq.). Le milieu est agité à 50°C pendant 2h et après retour à température ambiante, le milieu est filtré puis concentré à sec sous vide. Le produit est purifié sur gel de silice pour fournir le 6-Chloro-3-iodo-1H-pyrazolo[3,4-d]pyrimidin-4-ylamine (160 mg, 29%) (3) sous la forme d'un soldie blanc.

### c) 3-lodo-1-isopropyl-N⁶-methyl-1H-pyrazolo[3,4-d]pyrimidine-4,6-diamine

A une solution de 6-Chloro-3-iodo-1-isopropyl-1*H*-pyrazolo[3,4-*d*]pyrimidin-4-ylamine (150 mg; 0.44 mmol; 2eq.) (3) dans 1,4-dioxane (1 mL) est ajouté méthylamine dans le méthanol (555.47 µl; 2.00 M; 1.11 mmol; 5 eq.) et le tout est agité à 85°C. Après refroidissement du milieu, le tout est concentré sous vide. Le brut est chromatographié sur gel de silice (25 g, dépôt solide, éluant heptane/acétate d'éthyle de 45 à 85% d'acétate d'éthyle) pour fournir le 3-Iodo-1-isopropyl-*N*⁶-methyl-1*H*-pyrazolo[3,4-*d*]pyrimidine-4,6-diamine (65 mg; 88%) (4) sous la forme d'un solide blanc.

### d)3-(2-Amino-benzooxazol-5-yl)-1-isopropyl-N⁶-methyl-1H-pyrazolo[3,4-d] pyrimidine-4,6-diamine

A une solution de 3-lodo-1-isopropyl-*N*⁶-methyl-1*H*-pyrazolo[3,4-*d*]pyrimidine-4,6-diamine (65 mg; 0.20 mmol; 1 eq.) (4) est ajouté, 1,1'-bis(diphenylphosphino)ferrocenepalladium(II) dichloride, dichlorométhane (16 mg; 0.02 mmol; 0.10 eq.), 5-(4,4,5,5-tetramethyl-1,3,2-dioxaborolan-2-yl)benzo[d]oxazol-2-amine (76.35 mg; 0.29 mmol; 1.5 eq.) et une solution aqueuse 2M de potassium carbonate (0.29 mL; 2.00 M; 0.59 mmol; 3 eq.)dans le 1,4-dioxane (650.00 µl). Le milieu réactionnel est chauffé à 100°C pendant 1h. Après totale conversion, le milieu est refroidi puis concentré sous vide pour être directement chromatographié sur gel de silice (12 g, dépôt solide, éluant dichlorométhane/méthanol de 3 à 10% de méthanol). Le 3-(2-Amino-benzooxazol-5-yl)-1-isopropyl-*N*⁶-methyl-1*H*-pyrazolo[3,4-*d*]pyrimidine-4,6-diamine (40 mg; 59%) (5) est obtenu sous forme d'un solide ocre.

1H RMN (DMSO-d6) δ: 1.44 (d, J = 6.7 Hz, 6H), 2.82 (d, J = 4.7 Hz, 3H), 4.73 - 4.99 (m, 1H), 6.53 (s, 1H), 7.20 (dd, J = 8.2, 1.7 Hz, 1H), 7.37 (d, J = 1.7 Hz, 1H), 7.43 (d, J = 8.2 Hz, 1H), 7.51 (s, 2H)

MS (ESI) m/z = 339 [M+H] ⁺

### Exemple 27 : 5-(6-amino-4-methyl-1-neopentyl-1H-pyrazolo[3,4-d]pyrimidin-3-yl)benzo[d]oxazol-2-amine Ce composé peut être obtenu selon le procédé présenté dans la Figure 4.

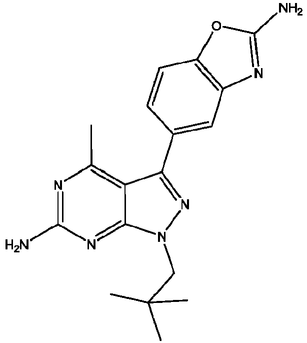

1H RMN (DMSO-d6) δ: 1H NMR (600 MHz, DMSO-d6, 300K) δ ppm 7.47 (s, 2 H) 7.36 - 7.43 (m, 2 H) 7.21 (dd, J=8.1, 1.4 Hz, 1 H) 6.72 (s, 2 H) 4.01 (s, 2 H) 2.36 (br s, 3 H) 0.97 (s, 9 H)

MS (ESI) m/z = 352 [M+H] ⁺

### Exemple 28 : 3-(2-cyclopropylamino-benzooxazol-5-yl)-1-isopropyl-1H-pyrazolo[3,4-d]pyrimidine-4,6-diamine

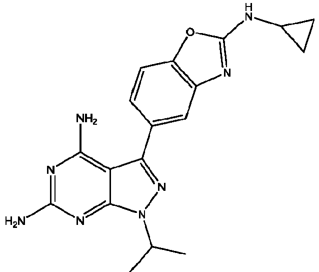

1H RMN (DMSO-d6) δ: 0.56 - 0.60 (m, 2 H), 0.73 - 0.77 (m, 2 H), 1.42 (d, J=6.7 Hz, 6 H), 2.76 (m, 1 H), 4.82 (spt, J=6.7 Hz, 1 H), 6.11 (br s, 4 H), 7.22 (dd, J=8.1, 1.8 Hz, 1 H), 7.43 (dd, J=1.8, 0.4 Hz, 1 H), 7.46 (dd, J=8.1, 0.4 Hz, 1 H), 8.26 (d, J=2.7 Hz, 1 H).

MS (ESI) m/z = 365 [M+H] ⁺

### Exemple 29: 3-(2-amino-7-methyl-benzooxazol-5-yl)-1-(2,2-dimethyl-butyl)-1H-pyrazolo[3,4-d]pyrimidine-4,6-diamine

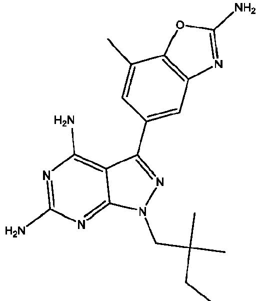

1H RMN (DMSO-d6) δ: 0.90 (d, J = 10.0 Hz, 9H), 1.32 (q, J = 7.5 Hz, 2H), 2.42 (s, 3H), 3.93 (s, 2H); 6.11 (s, 2H), 7.03 (s, 1H), 7.18 (d, J = 1.5 Hz, 1H), 7.47 (s, 2H) MS (ESI) m/z = 381 [M+H] ⁺

### Exemple 30: 3-(2-amino-benzooxazol-5-yl)-1-(1-methyl-cyclobutylmethyl)-1H-pyrazolo[3,4-d]pyrimidine-4,6-diamine

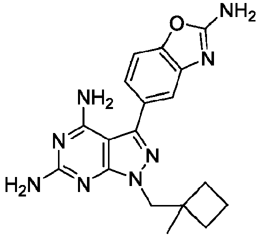

1H RMN (DMSO-d6) δ: 1.12 (s, 3H), 1.59 - 1.76 (m, 3H), 1.77 - 1.91 (m, 1H), 2.12 - 2.24 (m, 2H), 4.09 (s, 2H), 6.14 (s, 4H), 7.20 (dd, J = 8.2, 1.8 Hz, 1H), 7.37 (d, J = 1.7 Hz, 1H), 7.44 (d, J = 8.2 Hz, 1H), 7.50 (s, 2H).

MS (ESI) m/z = 365 [M+H] ⁺

### Exemple 31 : 5-(6-Amino-4-chloro-1-isopropyl-1H-pyrazolo[3,4-d]pyrimidin-3-yl)benzo[d]oxazol-2-amine

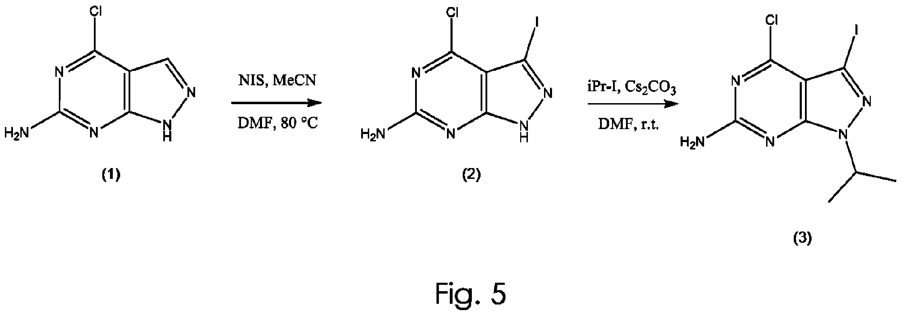

Ce composé peut être obtenu à partir du procédé présenté dans la Figure 5.

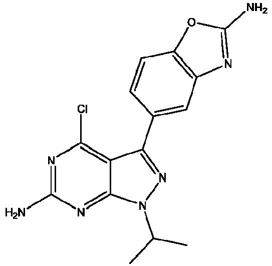

### a) 4-Chloro-3-iodo-1H-pyrazolo[3,4-d]pyrimidin-6-yl-amine

A une solution de 4-Chloro-1H-pyrazolo[3,4-d]pyrimidin-6-ylamine (4.00 g; 23.59 mmol; 1.00 eq.) (1) dans de l'acétonitrile (60.0 mL) et du DMF (4.00 mL), est ajouté le N-iodosuccinimide (10.61 g; 47.18 mmol; 2.00 eq.) à température ambiante. Le mélange est ensuite chauffé à 80°C pendant 5 jours. Le milieu réactionnel est concentré sous vide. Le milieu est dissout dans une solution de NaOH (1 M) et du MeTHF (30 mL). La phase aqueuse est extraite par MeTHF (5 mL). Les phases organiques sont combinées, séchées (MgSO₄) et concentrées et le résidu est trituré avec de l'Et₂O pour donner le 4-chloro-3-iodo-1H-pyrazolo[3,4-d]pyrimidin-6-yl-amine (4.52 g, 65%) (2) sous forme de solide beige.

1H RMN (DMSO-d₆) δ: 7.28-7.40 (br s, 2H), 13.5 (s, 1H)

MS (ESI) m/z = 295.77 [M+H]⁺

### b) 4-chloro-3-iodo-1-isopropyl-1H-pyrazolo[3,4-d]pyrimidin-6-yl-amine

4-Chloro-3-iodo-1H-pyrazolo[3,4-d]pyrimidin-6-yl-amine (2.54 g; 8.60 mmol; 1.00 eq.) (2) est dissout dans du DMF (50.0 mL) et du césium carbonate (2.80 g; 8.60 mmol; 1.00 eq.) est ajouté suivi par du 2-iodopropane (0.86 mL; 8.60 mmol; 1.00 eq.). Le mélange réactionnel est agité pendant 3h 30 à température ambiante. De l'eau est ajouté au milieu, qui est ensuite extrait par AcOEt. La phase organique recueillie est lavée, séchée (MgSO₄) et concentrée sous vide. Le produit brut est purifié par Prep LCMS (colonne C18, éluant : acétonitrile dans eau/0.1% d'acide formique.) pour donner le 4-chloro-3-iodo-1-isopropyl-1H-pyrazolo[3,4-d]pyrimidin-6-yl-amine (623.00 mg; 21.47 %) (3) sous forme de solide beige.

1H RMN (DMSO-d₆) δ: 1.40-1.42 (d, J = 6 Hz, 6H), 4.74-4.86 (h, J = 6 Hz, 1H), 7.39 (br s, 2H), 13.5 (s, 1H)

MS (ESI) m/z = 337.85 [M+H]⁺

### c) 5-(6-Amino-4-chloro-1-isopropyl-1H-pyrazolo[3,4-d]pyrimidin-3-yl)benzo[d]oxazol-2-amine

Dans un tube microonde de 6 mL, est ajouté du 4-chloro-3-iodo-1-isopropyl-1*H*-pyrazolo[3,4-d]pyrimidin-6-yl-amine (0.50 g; 1.48 mmol; 1.00 eq.), du 5-(4,4,5,5-tetramethyl-1,3,2-dioxaborolan-2-yl)benzo[d]oxazol-2-amine (0.58 g; 2.22 mmol; 1.50 eq.) dans de la 1,4-dioxanne (5.28 ml) suivi par une solution aqueuse de potassium carbonate (2.22 ml; 2.00 M; 4.44 mmol; 3.00 eq.) puis le mélange est dégazé sous azote trois fois et du 1,1'-bis(diphenylphosphino)ferrocenepalladium(II) dichloride dichloromethane (120.96 mg; 0.15 mmol; 0.10 eq.) est ensuite ajouté rapidement. Le milieu réactionnel est chauffé 1 heure à 100°C ou micro-onde, refroidi à température ambiante, dissout dans de l'AcOEt (10 mL), filtré et concentré à sec. Le résidu obtenu est purifié par Prep LCMS (colonne C18, éluant : acétonitrile dans eau/0.1% d'acide formique) pour donner le 5-(6-amino-4-chloro-1-isopropyl-1*H*-pyrazolo[3,4-d]pyrimidin-3-yl)benzo[d]oxazol-2-amine (0,21 g, 41%) sous la forme d'un solide blanc cristallin.

1H RMN (DMSO-d₆) δ: 1.47 (d, J = 6.7 Hz, 6H), 4.91 (p, J = 6.7 Hz, 1H), 7.22 - 7.32 (m, 3H), 7.41 (d, J = 8.2 Hz, 1H), 7.44 - 7.50 (m, 3H)

MS (ESI) m/z = 344 [M+H]⁺

### Exemple 32 : activités enzymatique mTOR et cellulaire mTORC1/mTORC2

### 32.1 Activité inhibitrice de la kinase mTOR

Le modèle de criblage de l'activité inhibitrice des molécules sur mTOR a été développé avec la technologie LANTHASCREEN^{™} (Lifetechnologies). Le substrat de la réaction (400 nM final), les dilutions sérielles des molécules (1% DMSO final) et l'enzyme (<1 nM) sont ajoutés successivement dans une plaque 384 puits (Corning 4514) dans un volume final de 10 µL par puits. Après 1 heure de réaction à température ambiante, 10 µL d'une solution contenant 10 mM final d'EDTA et 2 nM final d'anticorps marqués au Terbium sont ajoutés. Après ou moins 30 minutes d'incubation à température ambiante, le signal TR-FRET est mesuré avec un lecteur de microplaque adapté selon les recommandations du fournisseur. Les données sont normalisées par des contrôles positifs ('POS' contenant une concentration saturante d'inhibiteur de référence) et des contrôles négatifs ('NEG' contenant 1% DMSO) : % inhibition=((X-NEG)^{∗}100)/(POS-NEG). Les IC50 sont calculées à partir d'un modèle logistique à 4 paramètres à l'aide du logiciel XLFit (IDBS).

### 32.2 Activité inhibitrice mTORC1/mTORC2

Les cellules A431 sont ensemencées en milieu complet (DMEM+10% SVF) à 25 000 cellules par puits d'une plaque 96 puits coatés à la poly-L-Lysine. 24 heures avant l'expérience, le milieu est remplacé par du milieu sans sérum. Les dilutions sérielles des molécules à tester sont ajoutées (0.1% DMSO final). Après 3 heures d'incubation à 37°C, la phosphorylation des biomarqueurs S6RP (mTORC1) et AKT (mTORC2) est mesurée à l'aide de la technologie HTRF (Cisbio) selon les recommandations du fournisseur. Les données sont normalisées par des contrôles positifs ('POS' contenant une concentration saturante d'inhibiteur de référence) et des contrôles négatifs ('NEG' contenant 1% DMSO) : % inhibition=((X-NEG)^{∗}100)/(POS-NEG). Les IC50 sont calculées à partir d'un modèle logistique à 4 paramètres à l'aide du logiciel XLFit (IDBS).

Tableau des résultats d'activités :

| Exemple | Nom chimique | Ki mTOR (nM) | IC50 mTORC1 (nM) | IC50 mTORC2 (nM) |
|---|---|---|---|---|
| 1 | 3-(2-amino-benzooxazol-5-yl)-1-((S)-1,3-dimethyl-butyl)-1H-pyrazolo[3,4-d]pyrimidine-4,6-diamine | 0.8 | 1.0 | 1.5 |
| 2 | 3-(2-amino-6-fluoro-benzooxazol-5-yl)-1-((S)-1,3-dimethyl-butyl)-1H-pyrazolo[3,4-d]pyrimidine-4,6-diamine | 1.0 | 1.2 | 2.1 |
| 3 | 3-(2-amino-4-fluoro-benzooxazol-5-yl)-1-(1-methyl-cyclobutylmethyl)-1H-pyrazolo[3,4-d]pyrimidine-4,6-diamine | 0.3 | 0.2 | 1.0 |
| 4 | 3-(2-amino-6-fluoro-benzooxazol-5-yl)-1-(1-methyl-cyclobutylmethyl)-1H-pyrazolo[3,4-d]pyrimidine-4,6-diamine | 1.4 | 1.3 | 3.4 |
| 5 | 3-(2-amino-4-fluoro-benzooxazol-5-yl)-1-((S)-1,3-dimethyl-butyl)-1H-pyrazolo[3,4-d]pyrimidine-4,6-diamine | 0.4 | 0.5 | 1.1 |
| 6 | N-(5-(4,6-diamino-1-(2,2-dimethylbutyl)-1H-pyrazolo[3,4-d]pyrimidin-3-yl)benzo[d]oxazol-2-yl)acetamide | 0.5 | 0.7 | 5.9 |
| 7 | 3-(2-amino-benzooxazol-5-yl)-1-(2,2-dimethyl-propyl)-1H-pyrazolo[3,4-d]pyrimidine-4,6-diamine | 0.6 | 0.7 | 2.4 |
| 8 | 3-(2-amino-benzooxazol-5-yl)-1-(2,2-dimethyl-butyl)-1H-pyrazolo[3,4-d]pyrimidine-4,6-diamine | 0.6 | 1.2 | 3.0 |
| 9 | 3-(2-amino-benzooxazol-5-yl)-1-(3-methyl-thietan-3-ylmethyl)-1H-pyrazolo[3,4-d]pyrimidine-4,6-diamine | 0.6 | 1.0 | 2.8 |
| 10 | 3-(2-amino-4-fluoro-benzooxazol-5-yl)-1-(2,2-dimethyl-butyl)-1H-pyrazolo[3,4-d]pyrimidine-4,6-diamine | 0.8 | 0.2 | 1.2 |
| 11 | 1-(2,2-dimethyl-propyl)-3-(2-ethylamino-benzooxazol-5-yl)-1H-pyrazolo[3,4-d]pyrimidine-4,6-diamine | 0.8 | 5.0 | 5.9 |
| 12 | 3-(2-amino-benzooxazol-5-yl)-1-(2,2-dimethyl-but-3-enyl)-1H-pyrazolo[3,4-d]pyrimidine-4,6-diamine | 1.2 | 1.1 | 3.7 |
| 13 | (+)-3-(2-amino-benzooxazol-5-yl)-1-((S)-2-methyl-2-tetrahydro-furan-2-yl-propyl)-1H-pyrazolo[3,4-d] pyrimidine-4,6-diamine | 1.2 | 1.4 | 4.1 |
| 14 | 3-(2-amino-benzooxazol-5-yl)-1-(2,2-dimethyl-but-3-ynyl)-1H-pyrazolo[3,4-d]pyrimidine-4,6-diamine | 1.2 | 1.2 | 3.8 |
| 15 | 3-[4,6-diamino-3-(2-amino-benzooxazol-5-yl)-pyrazolo[3,4-d]pyrimidin-1-yl]-2,2-dimethyl-propan-1-ol | 1.5 | 1.7 | 7.2 |
| 16 | 3-(2-amino-benzooxazol-5-yl)-1-(2-methyl-2-methylsulfanyl-propyl)-1H-pyrazolo[3,4-d]pyrimidine-4,6-diamine | 1.7 | 2.2 | 10.2 |
| 17 | 3-(2-amino-4-chloro-benzooxazol-5-yl)-1-(2,2-dimethyl-propyl)-1H-pyrazolo[3,4-d]pyrimidine-4,6-diamine | 1.8 | 1.2 | 4.0 |
| 18 | 3-(2-amino-6-methoxy-benzooxazol-5-yl)-1-(2,2-dimethyl-propyl)-1H-pyrazolo[3,4-d]pyrimidine-4,6-diamine | 2.1 | 2.1 | 4.3 |
| 19 | 2-[4,6-diamino-1-(2,2-dimethyl-propyl)-1H-pyrazolo[3,4-d]pyrimidin-3-yl]-1H-pyrrolo[2,3-b]pyridin-5-ol | 2.3 | 27.4 | 65.5 |
| 20 | 3-(2-amino-6-methoxy-benzooxazol-5-yl)-1-(2,2-dimethyl-butyl)-1H-pyrazolo[3,4-d]pyrimidine-4,6-diamine | 2.9 | 2.1 | 7.0 |
| 21 | 3-(2-amino-benzooxazol-5-yl)-1-isopropyl-1H-pyrazolo[3,4-d] pyrimidine-4,6-diamine | 3.8 | 3.8 | 10.7 |
| 22 | 3-(2-amino-benzooxazol-5-yl)-1-(2-ethyl-2-methanesulfonyl-butyl)-1H-pyrazolo[3,4-d]pyrimidine-4,6-diamine | 3.9 | 10.2 | 26.6 |
| 23 | 3-(2-amino-benzooxazol-5-yl)-4-chloro-1-isobutyl-1H-pyrazolo[3,4-d] pyrimidin-6-ylamine | 5.8 | 2.8 | 5.9 |
| 24 | 3-(2-amino-benzooxazol-5-yl)-1-isopropyl-1H-pyrazolo[3,4-d] pyrimidin-6-ylamine | 6.1 | 6.8 | 15.5 |
| 25 | 3-(2-amino-6-chloro-benzooxazol-5-yl)-1-((S)-1,2-dimethyl-propyl)-1H-pyrazolo[3,4-d]pyrimidine-4,6-diamine | 8.1 | 4.5 | 13.1 |
| 26 | 3-(2-amino-benzooxazol-5-yl)-1-isopropyl-N^{∗}6^{∗}-methyl-1H-pyrazolo[3,4-d]pyrimidine-4,6-diamine | 13.4 | 29.7 | 62.2 |
| 27 | 5-(6-amino-4-methyl-1-neopentyl-1H-pyrazolo[3,4-d]pyrimidin-3-yl)benzo[d]oxazol-2-amine | 14.3 | 8.1 | 15.3 |
| 28 | 3-(2-cyclopropylamino-benzooxazol-5-yl)-1-isopropyl-1H-pyrazolo[3,4-d]pyrimidine-4,6-diamine | 21.0 | 43.8 | 40.2 |
| 29 | 3-(2-amino-7-methyl-benzooxazol-5-yl)-1-(2,2-dimethyl-butyl)-1H-pyrazolo[3,4-d]pyrimidine-4,6-diamine | 102.2 | 233.6 | 745.7 |
| 30 | 3-(2-amino-benzooxazol-5-yl)-1-(1-methyl-cyclobutylmethyl)-1H-pyrazolo[3,4-d]pyrimidine-4,6-diamine | 0.58 | 0.87 | 2.7 |
| 31 | 5-(6-Amino-4-chloro-1-isopropyl-1H-pyrazolo[3,4-d]pyrimidin-3-yl) benzo [d] oxazol-2-amine | 10 | 8.4 | 21 |

IC50 : concentration de l'inhibiteur causant 50% d'inhibition.

Il s'agit d'un indice pratique d'efficacité.

Ki : constante de dissociation du complexe enzyme - inhibiteur. Il indique l'affinité entre l'enzyme et l'inhibiteur (de façon inverse).

L'affinité d'un inhibiteur pour une enzyme est donnée par la constante d'inhibition Ki, qui représente la concentration en inhibiteur pour laquelle la moitié des sites enzymatiques sont occupés. Ainsi, l'affinité d'un inhibiteur est d'autant plus grande que le Ki est petit. Cette constante d'inhibition, exprimée en mole par litre correspond aussi à la constante de dissociation du complexe enzyme-inhibiteur.

Considérant les résultats ci-dessus, les composés avec des activités (IC50 mTORC1 et/ou IC50 mTORC2) inférieures à 5 nM sont plus particulièrement préférés.

### Exemple 33: Chronogramme d'un modèle de patch de dermatite atopique chez la souris

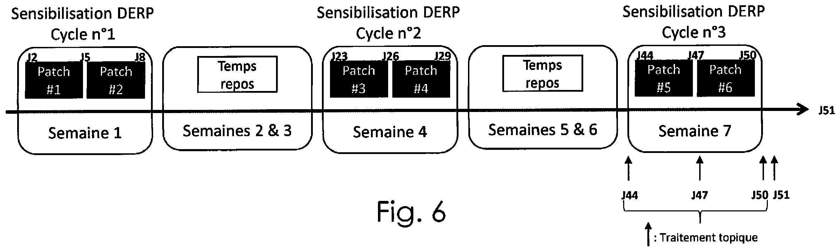

Tel qu'illustré par la Figure 6, des souris femelles Balb/c ont été traitées avec l'allergène Dermatophagoïdes pteronyssinus (DERP) au cours de 3 cycles de sensibilisation épicutanée (patch) sur la peau abdominale avec 2 patchs appliqués deux fois par semaine suivis de 2 semaines de repos, pendant 7 semaines. L'antagoniste (inhibiteur de mTOR) selon l'exemple 31 testé a été formulé à 0,07% dans un véhicule acétonique, appliqué sur la peau du dos de la souris (10 µl/oreille sur les deux faces) et laissé pendant 2 heures avant la mise en place des patchs DERP. Les traitements topiques avec l'antagoniste (inhibiteur de mTOR) ou un corticostéroïde (Bétaméthasone-17-Valérate à 0,01% utilisé comme contrôle) n'ont été effectués que 3 fois au cours de la 3ème et dernière semaine de sensibilisation (aux jours 44, 47 et 50).

### Exemple 34 : Test de perte d'eau trans-épidermique (ou TEWL) dans un modèle de patch de dermatite atopique après application d'un antagoniste de mTOR

Le protocole de traitement est illustré à la Figure 6.

Le taux de perte d'eau trans-épidermique TEWL est mesuré à l'aide d'un TEWAMETER^{®} sur un animal de contrôle. La sonde TEWAMETER^{®} mesure le gradient de densité de l'évaporation de l'eau de la peau indirectement à l'aide de deux paires de capteurs (température et humidité relative) à l'intérieur d'un cylindre creux. Un microprocesseur analyse les valeurs et exprime le taux d'évaporation en g/h/m2.

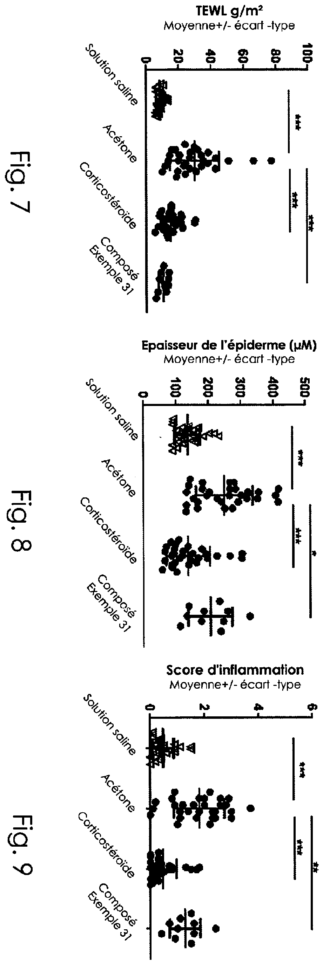

Les résultats obtenus sont illustrés à la Figure 7.

### Exemple 35 : Épaisseur de l'épiderme dans un modèle de patch de dermatite atopique après application d'un antagoniste de mTOR

Le protocole de traitement est illustré à la Figure 6.

L'épaisseur de l'épiderme est mesurée par analyses morphométriques histologiques sur des coupes de 6 µm d'épaisseur colorées à l'HE à partir d'échantillons de peau préalablement fixés au sérum physiologique.

Les résultats obtenus sont illustrés à la Figure 8.

### Exemple 36 : Score d'inflammation dans un modèle de patch de dermatite atopique après application d'un antagoniste de mTOR

Le protocole de traitement est illustré à la Figure 6.

Le score d'inflammation est défini visuellement en fonction d'échelles prédéfinies de sécheresse, éruption cutanée, calvitie, excoriation et lichénification. La moyenne des cinq scores attribués à une souris individuelle un jour donné donne le score clinique attribué le jour spécifique.

Les résultats obtenus sont illustrés à la Figure 9.

### Exemple 37 : Etude d'un antagoniste de mTOR selon l'invention dans un modèle de patch de dermatite atopique (AD)

Pour évaluer la contribution de mTOR à la réponse inflammatoire de la dermatite atopique, les inventeurs ont utilisé un modèle de dermatite atopique décrit précédemment basé sur des sensibilisations épicutanées répétées (Spergel et al. JCI 1998; Staumont-Sallé et al. JEM 2015), avec Dermatophagoïdes pteronyssinus (DERP) comme antigène. Dans ce modèle, les lésions cutanées provoquées par la dermatite atopique présentent une inflammation à dominante Th2 caractérisée par une infiltration dermique de lymphocytes T CD4+ et d'éosinophiles accompagnée du dépôt de produits éosinophiles et d'une augmentation de l'expression cutanée des cytokines Th2.

Suivant le protocole de l'exemple 33, la réponse inflammatoire de la dermatite atopique a été provoquée par une sensibilisation épicutanée avec des patchs imbibés de 100 µg d'allergène dans une solution saline stérile (Dermatophagoïdes pteronyssinus ou DERP) ou avec un véhicule appliqués sur la peau abdominale 24h après le rasage et laissés en place pendant trois périodes d'une semaine (avec renouvellement du patch en milieu de semaine), avec un intervalle de 2 semaines entre les applications. La présence des patchs et d'un pansement occlusif transparent par-dessus, a empêché le léchage, les morsures et les éraflures chez la souris; par conséquent, la réponse inflammatoire dans ce modèle n'est pas due au cycle des démangeaisons. L'antagoniste selon l'exemple 31 (inhibiteur de mTOR) a été formulé à 0,07% dans un véhicule à base d'acétone, appliqué sur la peau sensibilisée 2 heures avant l'installation des patchs DERP. Les traitements topiques avec l'antagoniste selon l'invention ou un corticostéroïde (utilisé comme témoin) n'ont été pratiqués que 3 fois au cours de la 3ème et dernière semaine de sensibilisation (aux jours 44, 47 et 50).

Au moment du retrait du dernier patch (jour 51), des échantillons de peau ont été collectés pour des analyses histologiques et immunohistologiques. La Figure 6 indique le chronogramme des traitements.

Les résultats sont présentés aux Figures 7 à 9. L'antagoniste selon l'exemple 31 (inhibiteur de mTOR) diminue tous les paramètres étudiés.

L'augmentation du taux de perte d'eau trans-épidermique TEWL, un signe clinique de dysfonctionnement de la barrière cutanée, est significativement diminuée (-68%).

Un autre paramètre de dysfonctionnement de la barrière cutanée, l'acanthose de l'épiderme, est partiellement restaurée avec l'antagoniste de mTOR (-39% respectivement sur l'épaisseur de l'épiderme).

Les paramètres inflammatoires sont également impactés tels que les scores inflammatoires (-40).

L'efficacité d'un antagoniste de mTOR (inhibiteur de mTOR) selon l'invention est assez proche du traitement par corticostéroïde.

De manière surprenante, la Demanderesse a maintenant découvert que, dans un modèle murin de dermatite atopique qui n'est pas due à des démangeaisons, les antagonistes mTOR (inhibiteurs de mTOR) diminuent significativement les signes cliniques (tels que les scores cliniques et la perte d'eau transépidermique ou TEWL) et les paramètres histologiques (tels que le nombre de cellules inflammatoires dans le derme et l'épaisseur de l'épiderme).

## Revendications

1. Composé de formule générale (I) 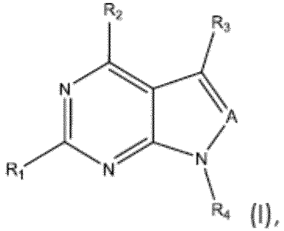 ou un de ses sels pharmaceutiquement acceptables, dans laquelle:
R₁ représente un radical -NH₂;
R₂ représente un atome d'halogène choisi parmi F, Cl, Br et I, ou un radical -NH₂;
R₃ représente un radical hétéroaromatique bicycle fusionné, non substitué ou mono-ou polysubstitué par un ou plusieurs radical(aux) choisi(s) parmi un atome d'halogène choisi parmi F, Cl, Br et I, -NHRs, nitrile, méthyle, éthyle, trifluorométhyle, -ORs;
avec R₅ représentant un atome d'hydrogène, un radical cyclopropyle, acyle, alkyle en C₁-C₆ saturé ou insaturé, interrompu ou non par un hétéroatome O, S, et non substitué ou substitué par un cycloalkyle ou un hétérocycloalkyle en C₃-C₅; et
avec R₆ représentant un atome d'hydrogène ou un radical méthyle;
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆; et
A représente un atome d'azote.

2. Composé de formule générale (I) selon la revendication 1, ou un de ses sels pharmaceutiquement acceptables, dans laquelle:
R₁ représente un radical -NH₂;
R₂ représente un atome CI ou un radical -NH₂;
R₃ représente un radical hétéroaromatique bicycle fusionné, mono- ou polysubstitué par un ou plusieurs radical(aux) choisi(s) parmi un atome d'halogène choisi parmi F, Cl, Br et I, -NHRs, nitrile, méthyle, éthyle, trifluorométhyle, -ORs;
avec R₅ représentant un atome d'hydrogène, un radical cyclopropyle, acyle, alkyle en C₁-C₆ saturé ou insaturé, interrompu ou non par un hétéroatome O, S, et non substitué ou substitué par un cycloalkyle ou un hétérocycloalkyle en C₃-C₅; et
avec R₆ représentant un atome d'hydrogène ou un radical méthyle;
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆; et
A représente un atome d'azote.

3. Composé selon la revendication 2, de formule (la) 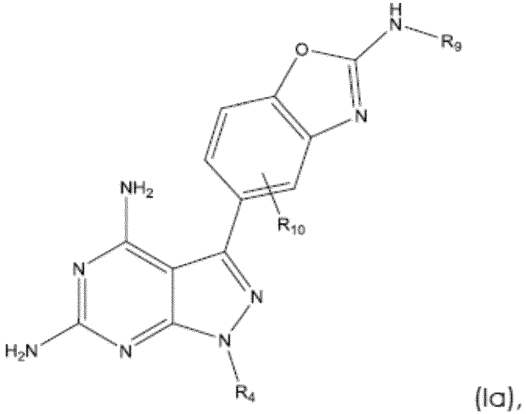 ou un de ses sels pharmaceutiquement acceptables, dans laquelle:
R₄ représente un radical alkyle en C₃-C₁₀ ramifié, un cycle ou bicycle en C₃-C₁₀, interrompu ou non par un hétéroatome O, S, et non substitué ou substitué par un ou plusieurs radical(aux) choisi(s) parmi fluoro, cycloalkyle ou hétérocycloalkyle en C₃-C₆;
R₉ représente un atome d'hydrogène, ou un radical méthyle, éthyle; et
un ou plusieurs R₁₀, pris indépendamment, représentent un atome d'hydrogène, un atome d'halogène choisi parmi Cl, F, ou un radical -OH, méthyle, -OMe.

4. Composé de formule (la) selon la revendication 3, ou un de ses sels pharmaceutiquement acceptables, dans laquelle:
R₄ représente un alkyle en C₃-C₁₀ ramifié;
R₉ représente un atome d'hydrogène; et
un ou plusieurs R₁₀, pris indépendamment, représentent un atome d'hydrogène, un atome d'halogène choisi parmi Cl, F, ou un radical méthyle.

5. Composé de formule (la) selon la revendication 4, ou un de ses sels pharmaceutiquement acceptables, dans laquelle:
R₄ représente un alkyle en C₄-C₆ ramifié;
R₉ représente un atome d'hydrogène; et
R₁₀ représente un atome d'hydrogène ou de fluor.

6. Composé inhibiteur de mTOR de formule (I) selon l'une des revendications 1 à 5, choisi parmi les composés suivants:
• 3-(2-amino-benzooxazol-5-yl)-1-((S)-1,3-dimethyl-butyl)-1H-pyrazolo[3,4-d]pyrimidine-4,6-diamine;
• 3-(2-amino-6-fluoro-benzooxazol-5-yl)-1-((S)-1,3-dimethyl-butyl)-1H-pyrazolo[3,4-d]pyrimidine-4,6-diamine;
• 3-(2-amino-4-fluoro-benzooxazol-5-yl)-1-(1-methyl-cyclobutylmethyl)-1H-pyrazolo[3,4-d]pyrimidine-4,6-diamine;
• 3-(2-amino-6-fluoro-benzooxazol-5-yl)-1-(1-methyl-cyclobutylmethyl)-1H-pyrazolo[3,4-d]pyrimidine-4,6-diamine;
• 3-(2-amino-4-fluoro-benzooxazol-5-yl)-1-((S)-1,3-dimethyl-butyl)-1H-pyrazolo[3,4-d]pyrimidine-4,6-diamine;
• N-(5-(4,6-diamino-1-(2,2-dimethylbutyl)-1H-pyrazolo[3,4-d]pyrimidin-3-yl)benzo[d]oxazol-2-yl)acetamide;
• 3-(2-amino-benzooxazol-5-yl)-1-(2,2-dimethyl-propyl)-1H-pyrazolo[3,4-d]pyrimidine-4,6-diamine;
• 3-(2-amino-benzooxazol-5-yl)-1-(2,2-dimethyl-butyl)-1H-pyrazolo[3,4-d]pyrimidine-4,6-diamine;
• 3-(2-amino-benzooxazol-5-yl)-1-(3-methyl-thietan-3-ylmethyl)-1H-pyrazolo[3,4-d]pyrimidine-4,6-diamine;
• 3-(2-amino-4-fluoro-benzooxazol-5-yl)-1-(2,2-dimethyl-butyl)-1H-pyrazolo[3,4-d]pyrimidine-4,6-diamine;
• 1-(2,2-dimethyl-propyl)-3-(2-ethylamino-benzooxazol-5-yl)-1H-pyrazolo[3,4-d]pyrimidine-4,6-diamine;
• 3-(2-amino-benzooxazol-5-yl)-1-(2,2-dimethyl-but-3-enyl)-1H-pyrazolo[3,4-d]pyrimidine-4,6-diamine;
• (+)-3-(2-amino-benzooxazol-5-yl)-1-((S)-2-methyl-2-tetrahydro-furan-2-yl-propyl)-1H-pyrazolo[3,4-d]pyrimidine-4,6-diamine;
• 3-(2-amino-benzooxazol-5-yl)-1-(2,2-dimethyl-but-3-ynyl)-1H-pyrazolo[3,4-d]pyrimidine-4,6-diamine;
• 3-[4,6-diamino-3-(2-amino-benzooxazol-5-yl)-pyrazolo[3,4-d]pyrimidin-1-yl]-2,2-dimethyl-propan-1-ol;
• 3-(2-amino-benzooxazol-5-yl)-1-(2-methyl-2-methylsulfanyl-propyl)-1H-pyrazolo[3,4-d]pyrimidine-4,6-diamine;
• 3-(2-amino-4-chloro-benzooxazol-5-yl)-1-(2,2-dimethyl-propyl)-1H-pyrazolo[3,4-d]pyrimidine-4,6-diamine;
• 3-(2-amino-6-methoxy-benzooxazol-5-yl)-1-(2,2-dimethyl-propyl)-1H-pyrazolo[3,4-d]pyrimidine-4,6-diamine;
• 2-[4,6-diamino-1-(2,2-dimethyl-propyl)-1H-pyrazolo[3,4-d]pyrimidin-3-yl]-1H-pyrrolo[2,3-b]pyridin-5-ol;
• 3-(2-amino-6-methoxy-benzooxazol-5-yl)-1-(2,2-dimethyl-butyl)-1H-pyrazolo[3,4-d]pyrimidine-4,6-diamine;
• 3-(2-amino-benzooxazol-5-yl)-1-isopropyl-1H-pyrazolo[3,4-d]pyrimidine-4,6-diamine;
• 3-(2-amino-benzooxazol-5-yl)-1-(2-ethyl-2-methanesulfonyl-butyl)-1H-pyrazolo[3,4-d]pyrimidine-4,6-diamine;
• 3-(2-amino-benzooxazol-5-yl)-4-chloro-1-isobutyl-1H-pyrazolo[3,4-d]pyrimidin-6-ylamine;
• 3-(2-amino-benzooxazol-5-yl)-1-isopropyl-1H-pyrazolo[3,4-d]pyrimidin-6-ylamine;
• 3-(2-amino-6-chloro-benzooxazol-5-yl)-1-((S)-1,2-dimethyl-propyl)-1H-pyrazolo[3,4-d]pyrimidine-4,6-diamine;
• 3-(2-Amino-benzooxazol-5-yl)-1-isopropyl-N*6*-methyl-1H-pyrazolo[3,4-d]pyrimidine-4,6-diamine;
• 5-(6-amino-4-methyl-1-neopentyl-1H-pyrazolo[3,4-d]pyrimidin-3-yl)benzo[d]oxazol-2-amine;
• 3-(2-cyclopropylamino-benzooxazol-5-yl)-1-isopropyl-1H-pyrazolo[3,4-d]pyrimidine-4,6-diamine;
• 3-(2-amino-7-methyl-benzooxazol-5-yl)-1-(2,2-dimethyl-butyl)-1H-pyrazolo[3,4-d]pyrimidine-4,6-diamine;
• 3-(2-amino-benzooxazol-5-yl)-1-(1-methyl-cyclobutylmethyl)-1H-pyrazolo[3,4-d]pyrimidine-4,6-diamine;
• 5-(6-Amino-4-chloro-1-isopropyl-1*H*-pyrazolo[3,4-d]pyrimidin-3-yl)benzo[d]oxazol-2-amine;
ou un de ses sels pharmaceutiquement acceptables.

7. Composition comprenant, dans un milieu physiologiquement acceptable, un composé selon l'une des revendications 1 à 6 ou un de ses sels pharmaceutiquement acceptables.

8. Composition selon la revendication 7, **caractérisée en ce qu'**elle comprend entre 0,001% et 5% dudit composé en poids du poids total de la composition.

9. Composition selon la revendication 7 ou 8, **caractérisée en ce qu'**elle est sous une forme adaptée pour une administration par voie orale ou topique.

10. Composition selon la revendication 9, **caractérisée en ce qu'**elle est sous une forme adaptée pour une administration par voie topique.

11. Composition selon l'une des revendications 7 à 10, pour son utilisation en tant que médicament.

12. Composition selon la revendication 11, pour son utilisation dans le traitement des affections dermatologiques liées à un trouble de la kératinisation avec une composante proliférative, inflammatoire et/ou immuno-allergique telles que le psoriasis, la dermatite atopique, la kératose actinique, ou l'acné.

13. Composition selon la revendication 12, pour son utilisation dans le traitement de la dermatite atopique.

14. Composition selon la revendication 13, pour son utilisation dans le traitement de la composante inflammatoire de la dermatite atopique.

15. Composition selon la revendication 13, pour son utilisation pour renforcer la fonction de barrière cutanée chez un patient souffrant de dermatite atopique.

## Patentansprüche

1. Verbindung der allgemeinen Formel (I) 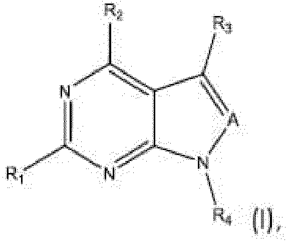 oder ein pharmazeutisch verträgliches Salz davon, wobei:
R₁ einen -NH₂-Rest darstellt;
R₂ ein Halogenatom, das aus F, Cl, Br und I ausgewählt ist, oder einen -NH₂-Rest darstellt;
R₃ einen kondensierten heteroaromatischen bizyklischen Rest darstellt, der nicht substituiert oder durch einen oder mehrere Rest(e) mono- oder polysubstituiert ist,
die aus einem Halogenatom ausgewählt sind, das aus F, Cl, Br und I, -NHR₅, Nitril, Methyl, Äthyl, Trifluormethyl, -ORe ausgewählt ist;
wobei R₅ ein Wasserstoffatom, einen Cyclopropyl-, Acyl-, gesättigten oder
ungesättigten C₁-C₆-Alkylrest darstellt, der durch ein O-, S-Heteroatom unterbrochen ist oder nicht und nicht substituiert oder durch ein C₃-C₅-Cycloalkyl oder - Heterocycloalkyl substituiert ist; und
wobei R₆ ein Wasserstoffatom oder einen Methylrest darstellt;
R₄ einen verzweigten C₃-C₁₀-Alkylrest, einen C₃-C₁₀-Zyklus oder -Bizyklus darstellt,
der durch ein O-, S-Heteroatom unterbrochen ist oder nicht und nicht substituiert oder durch einen oder mehrere Rest(e) substituiert ist, die aus Fluor, C₃-C₆-Cycloalkyl oder -Heterocycloalkyl ausgewählt sind; und
A ein Stickstoffatom darstellt.

2. Verbindung der allgemeinen Formel (I) nach Anspruch 1 oder ein pharmazeutisch verträgliches Salz davon, wobei:
R₁ einen -NH₂-Rest darstellt;
R₂ ein CI-Atom oder einen -NH₂-Rest darstellt;
R₃ einen kondensierten heteroaromatischen bizyklischen Rest darstellt, der durch einen oder mehrere Rest(e) mono- oder polysubstituiert ist, die aus einem Halogenatom ausgewählt sind, das aus F, Cl, Br und I, -NHR₅, Nitril, Methyl, Äthyl, Trifluormethyl, -ORe ausgewählt ist;
wobei R₅ ein Wasserstoffatom, einen Cyclopropyl-, Acyl-, gesättigten oder ungesättigten C₁-C₆-Alkylrest darstellt, der durch ein O-, S-Heteroatom unterbrochen ist oder nicht und nicht substituiert oder durch ein C₃-C₅-Cycloalkyl oder - Heterocycloalkyl substituiert ist; und
wobei R₆ ein Wasserstoffatom oder einen Methylrest darstellt;
R₄ einen verzweigten C₃-C₁₀-Alkylrest, einen C₃-C₁₀-Zyklus oder -Bizyklus darstellt, der durch ein O-, S-Heteroatom unterbrochen ist oder nicht und nicht substituiert oder durch einen oder mehrere Rest(e) substituiert ist, die aus Fluor, C₃-C₆-Cycloalkyl oder -Heterocycloalkyl ausgewählt sind; und
A ein Stickstoffatom darstellt.

3. Verbindung nach Anspruch 2 mit der folgenden Formel (la) 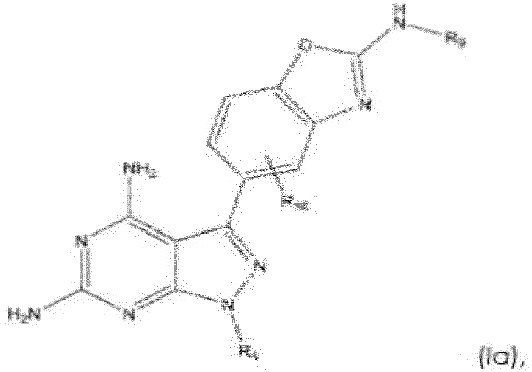 oder ein pharmazeutisch verträgliches Salz davon, wobei:
R₄ einen verzweigten C₃-C₁₀-Alkylrest, einen C₃-C₁₀-Zyklus oder -Bizyklus darstellt,
der durch ein O-, S-Heteroatom unterbrochen ist oder nicht und nicht substituiert oder durch einen oder mehrere Rest(e) substituiert ist, die aus Fluor, C₃-C₆-Cycloalkyl oder -Heterocycloalkyl ausgewählt sind;
R₉ ein Wasserstoffatom oder einen Methyl-, Äthylrest darstellt; und
ein oder mehrere R₁₀ unabhängig voneinander ein Wasserstoffatom, ein Halogenatom, das aus Cl, F ausgewählt ist, oder einen -OH-, Methyl-, -OMe-Rest darstellen.

4. Verbindung der Formel (la) nach Anspruch 3 oder ein pharmazeutisch verträgliches Salz davon, wobei:
R₄ ein verzweigtes C₃-C₁₀-Alkyl darstellt;
R₉ ein Wasserstoffatom darstellt; und
ein oder mehrere R₁₀ unabhängig voneinander ein Wasserstoffatom, ein Halogenatom, das aus Cl, F ausgewählt ist, oder einen Methylrest darstellen.

5. Verbindung der Formel (la) nach Anspruch 4 oder ein pharmazeutisch verträgliches Salz davon, wobei:
R₄ ein verzweigtes C₄-C₆-Alkyl darstellt;
R₉ ein Wasserstoffatom darstellt; und
R₁₀ ein Wasserstoff- oder Fluoratom darstellt.

6. mTOR-Inhibitor-Verbindung der Formel (I) nach einem der Ansprüche 1 bis 5, ausgewählt aus den folgenden Verbindungen:
• 3-(2-amino-benzooxazol-5-yl)-1-((S)-1,3-dimethyl-butyl)-1H-pyrazolo[3,4-d]pyrimidin-4,6-diamin;
• 3-(2-amino-6-fluor-benzooxazol-5-yl)-1-((S)-1,3-dimethyl-butyl)-1H-pyrazolo[3,4-d]pyrimidin-4,6-diamin;
• 3-(2-amino-4-fluor-benzooxazol-5-yl)-1-(1-methyl-cyclobutylmethyl)-1H-pyrazolo[3,4-d]pyrimidin-4,6-diamin;
• 3-(2-amino-6-fluor-benzooxazol-5-yl)-1-(1-methyl-cyclobutylmethyl)-1H-pyrazolo[3,4-d]pyrimidin-4,6-diamin;
• 3-(2-amino-4-fluor-benzooxazol-5-yl)-1 -((S)-1,3-dimethyl-butyl)-1H-pyrazolo[3,4-d]pyrimidin-4,6-diamin;
• N-(5-(4,6-diamino-1-(2,2-dimethylbutyl)-1H-pyrazolo[3,4-d]pyrimidin-3-yl)benzo[d]oxazol-2-yl)acetamid;
• 3-(2-amino-benzooxazol-5-yl)-1-(2,2-dimethyl-propyl)-1H-pyrazolo[3,4-d]pyrimidin-4,6-diamin;
• 3-(2-amino-benzooxazol-5-yl)-1-(2,2-dimethyl-butyl)-1H-pyrazolo[3,4-d]pyrimidin-4,6-diamin;
• 3-(2-amino-benzooxazol-5-yl)-1-(3-methyl-thietan-3-ylmethyl)-1H-pyrazolo[3,4-d]pyrimidin-4,6-diamin;
• 3-(2-amino-4-fluor-benzooxazol-5-yl)-1-(2,2-dimethyl-butyl)-1H-pyrazolo[3,4-d]pyrimidin-4,6-diamin;
• 1-(2,2-dimethyl-propyl)-3-(2-ethylamino-benzooxazol-5-yl)-1H-pyrazolo[3,4-d]pyrimidin-4,6-diamin;
• 3-(2-amino-benzooxazol-5-yl)-1-(2,2-dimethyl-but-3-enyl)-1H-pyrazolo[3,4-d]pyrimidin-4,6-diamin;
• (+)-3-(2-amino-benzooxazol-5-yl)-1-((S)-2-methyl-2-tetrahydro-furan-2-yl-propyl)-1H-pyrazolo[3,4-d]pyrimidin-4,6-diamin;
• 3-(2-amino-benzooxazol-5-yl)-1-(2,2-dimethyl-but-3-ynyl)-1H-pyrazolo[3,4-d]pyrimidin-4,6-diamin;
• 3-[4,6-diamino-3-(2-amino-benzooxazol-5-yl)-pyrazolo[3,4-d]pyrimidin-1-yl]-2,2-dimethyl-propan-1 -ol;
• 3-(2-amino-benzooxazol-5-yl)-1-(2-methyl-2-methylsulfanyl-propyl)-1H-pyrazolo[3,4-d]pyrimidin-4,6-diamin;
• 3-(2-amino-4-chlor-benzooxazol-5-yl)-1-(2,2-dimethyl-propyl)-1H-pyrazolo[3,4-d]pyrimidin-4,6-diamin;
• 3-(2-amino-6-methoxy-benzooxazol-5-yl)-1-(2,2-dimethyl-propyl)-1H-pyrazolo[3,4-d]pyrimidin-4,6-diamin;
• 2-[4,6-diamino-1-(2,2-dimethyl-propyl)-1H-pyrazolo[3,4-d]pyrimidin-3-yl]-1H-pyrrolo[2,3-b]pyridin-5-ol;
• 3-(2-amino-6-methoxy-benzooxazol-5-yl)-1-(2,2-dimethyl-butyl)-1H-pyrazolo[3,4-d]pyrimidin-4,6-diamin;
• 3-(2-amino-benzooxazol-5-yl)-1-isopropyl-1H-pyrazolo[3,4-d]pyrimidin-4,6-diamin;
• 3-(2-amino-benzooxazol-5-yl)-1-(2-ethyl-2-methansulfonyl-butyl)-1H-pyrazolo[3,4-d]pyrimidin-4,6-diamin;
• 3-(2-amino-benzooxazol-5-yl)-4-chlor-1-isobutyl-1H-pyrazolo[3,4-d]pyrimidin-6-ylamin;
• 3-(2-amino-benzooxazol-5-yl)-1-isopropyl-1H-pyrazolo[3,4-d]pyrimidin-6-ylamin;
• 3-(2-amino-6-chlor-benzooxazol-5-yl)-1-((S)-1,2-dimethyl-propyl)-1H-pyrazolo[3,4-d]pyrimidin-4,6-diamin;
• 3-(2-Amino-benzooxazol-5-yl)-1-isopropyl-N*6*-methyl-1H-pyrazolo[3,4-d]pyrimidin-4,6-diamin;
• 5-(6-amino-4-methyl-1-neopentyl-1H-pyrazolo[3,4-d]pyrimidin-3-yl)benzo[d]oxazol-2-amin;
• 3-(2-cyclopropylamino-benzooxazol-5-yl)-1-isopropyl-1H-pyrazolo[3,4-d]pyrimidin-4,6-diamin;
• 3-(2-amino-7-methyl-benzooxazol-5-yl)-1-(2,2-dimethyl-butyl)-1H-pyrazolo[3,4-d]pyrimidin-4,6-diamin;
• 3-(2-amino-benzooxazol-5-yl)-1-(1-methyl-cyclobutylmethyl)-1H-pyrazolo[3,4-d]pyrimidin-4,6-diamin;
• 5-(6-Amino-4-chlor-1-isopropyl-1*H*-pyrazolo[3,4-d]pyrimidin-3-yl)benzo[d]oxazol-2-amin;
oder ein pharmazeutisch verträgliches Salz davon.

7. Zusammensetzung, die in einem physiologisch annehmbaren Medium eine Verbindung nach einem der Ansprüche 1 bis 6 oder ein pharmazeutisch verträgliches Salz davon umfasst.

8. Zusammensetzung nach Anspruch 7, **dadurch gekennzeichnet, dass** sie zwischen 0,001 Gew.-% und 5 Gew.-% der besagten Verbindung des Gesamtgewichts der Zusammensetzung umfasst.

9. Zusammensetzung nach Anspruch 7 oder 8, **dadurch gekennzeichnet, dass** sie in einer Form vorliegt, die für eine orale oder topische Verabreichung geeignet ist.

10. Zusammensetzung nach Anspruch 9, **dadurch gekennzeichnet, dass** sie in einer Form vorliegt, die für eine topische Verabreichung geeignet ist.

11. Zusammensetzung nach einem der Ansprüche 7 bis 10 zu ihrer Verwendung als Medikament.

12. Zusammensetzung nach Anspruch 11 zu ihrer Verwendung bei der Behandlung von dermatologischen Erkrankungen, die mit einer Keratinisierungsstörung mit einer proliferativen, entzündlichen und/oder immunallergischen Komponente wie die Psoriasis, atopische Dermatitis, aktinische Keratose oder Akne verbunden sind.

13. Zusammensetzung nach Anspruch 12 zu ihrer Verwendung bei der Behandlung der atopischen Dermatitis.

14. Zusammensetzung nach Anspruch 13 zu ihrer Verwendung bei der Behandlung der entzündlichen Komponente der atopischen Dermatitis.

15. Zusammensetzung nach Anspruch 13 zu ihrer Verwendung zum Stärken der Barrierefunktion der Haut bei einem Patienten, der an atopischer Dermatitis leidet.

## Claims

1. Compound of general formula (I) 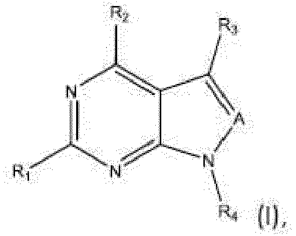 or a pharmaceutically acceptable salt thereof, wherein:
R₁ represents a -NH₂ radical;
R₂ represents a halogen atom selected from F, CI, Br and I, or an -NH₂ radical;
R₃ represents a fused bicyclic heteroaromatic radical, unsubstituted or mono- or polysubstituted with one or more radicals selected from a halogen atom selected from F, CI, Br and I, -NHR₅, nitrile, methyl, ethyl, trifluoromethyl, -OR₆;
with R₅ representing a hydrogen atom, a saturated or unsaturated C₁-C₆ cyclopropyl, acyl, alkyl radical, interrupted or not by an O, S heteroatom, and unsubstituted or substituted with a C₃-C₅ cycloalkyl or heterocycloalkyl; and
with R₆ representing a hydrogen atom or a methyl radical;
R₄ represents a branched C₃-C₁₀ alkyl radical, a C₃-C₁₀ cycle or bicycle, interrupted or not by an O, S heteroatom, and unsubstituted or substituted with one or more radical(s) selected from fluoro, C₃-C₆ cycloalkyl or heterocycloalkyl; and
A represents a nitrogen atom.

2. Compound of general formula (I) according to claim 1, or a pharmaceutically acceptable salt thereof, wherein:
R₁ represents a -NH₂ radical;
R₂ represents a CI atom or an -NH₂ radical;
R₃ represents a fused bicyclic heteroaromatic radical, unsubstituted or mono- or
polysubstituted with one or more radicals selected from a halogen atom selected from F, CI, Br and I, -NHR₅, nitrile, methyl, ethyl, trifluoromethyl, -OR₆;
with R₅ representing a hydrogen atom, a saturated or unsaturated C₁-C₆ cyclopropyl, acyl, alkyl radical, interrupted or not by an O, S heteroatom, and unsubstituted or substituted with a C₃-C₅ cycloalkyl or heterocycloalkyl; and
with R₆ representing a hydrogen atom or a methyl radical;
R₄ represents a branched C₃-C₁₀ alkyl radical, a C₃-C₁₀ cycle or bicycle, interrupted or not by an O, S heteroatom, and unsubstituted or substituted with one or more radical(s) selected from fluoro, C₃-C₆ cycloalkyl or heterocycloalkyl; and
A represents a nitrogen atom.

3. Compound according to claim 2, of formula (la) 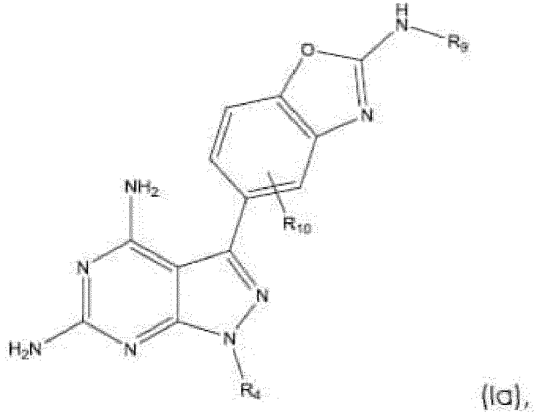 or a pharmaceutically acceptable salt thereof, wherein:
R₄ represents a branched C₃-C₁₀ alkyl radical, a C₃-C₁₀ cycle or bicycle, interrupted or not by an O, S heteroatom, and unsubstituted or substituted with one or more radical(s) selected from fluoro, C₃-C₆ cycloalkyl or heterocycloalkyl;
R₉ represents a hydrogen atom, or a methyl or ethyl radical; and
one or more R₁₀, taken independently, represent a hydrogen atom, a halogen atom selected from CI, F, or an -OH, methyl or -OMe radical.

4. Compound of formula (Ia) according to claim 3, or a pharmaceutically acceptable salt thereof, wherein:
R₄ represents a branched C₃-C₁₀ alkyl;
R₉ represents a hydrogen atom; and
one or more R₁₀, taken independently, represent a hydrogen atom, a halogen atom selected from Cl, F, or a methyl radical.

5. Compound of formula (Ia) according to claim 4, or a pharmaceutically acceptable salt thereof, wherein:
R₄ represents a branched C₃-C₁₀ alkyl;
R₉ represents a hydrogen atom; and
R₁₀ represents a hydrogen or fluorine atom.

6. mTOR inhibitor compound of formula (I) according to one of claims 1 to 5, selected from the following compounds:
• 3-(2-amino-benzooxazol-5-yl)-1-((S)-1,3-dimethyl-butyl)-1H-pyrazolo[3,4-d]pyrimidine-4,6-diamine;
• 3-(2-amino-6-fluoro-benzooxazol-5-yl)-1-((S)-1,3-dimethyl-butyl)-1H-pyrazolo[3,4-d]pyrimidine-4,6-diamine;
• 3-(2-amino-4-fluoro-benzooxazol-5-yl)-1-(1-methyl-cyclobutylmethyl)-1H-pyrazolo[3,4-d]pyrimidine-4,6-diamine;
• 3-(2-amino-6-fluoro-benzooxazol-5-yl)-1-(1-methyl-cyclobutylmethyl)-1H-pyrazolo[3,4-d]pyrimidine-4,6-diamine;
• 3-(2-amino-4-fluoro-benzooxazol-5-yl)-1-((S)-1,3-dimethyl-butyl)-1H-pyrazolo[3,4-d]pyrimidine-4,6-diamine;
• N-(5-(4,6-diamino-1-(2,2-dimethylbutyl)-1H-pyrazolo[3,4-d]pyrimidin-3-yl)benzo[d]oxazol-2-yl)acetamide;
• 3-(2-amino-benzooxazol-5-yl)-1-(2,2-dimethyl-propyl)-1H-pyrazolo[3,4-d]pyrimidine-4,6-diamine;
• 3-(2-amino-benzooxazol-5-yl)-1-(2,2-dimethyl-butyl)-1H-pyrazolo[3,4-d]pyrimidine-4,6-diamine;
• 3-(2-amino-benzooxazol-5-yl)-1-(3-methyl-thietan-3-ylmethyl)-1H-pyrazolo[3,4-d]pyrimidine-4,6-diamine;
• 3-(2-amino-4-fluoro-benzooxazol-5-yl)-1-(2,2-dimethyl-butyl)-1H-pyrazolo[3,4-d]pyrimidine-4,6-diamine;
• 1-(2,2-dimethyl-propyl)-3-(2-ethylamino-benzooxazol-5-yl)-1H-pyrazolo[3,4-d]pyrimidine-4,6-diamine;
• 3-(2-amino-benzooxazol-5-yl)-1-(2,2-dimethyl-but-3-enyl)-1H-pyrazolo[3,4-d]pyrimidine-4,6-diamine;
• (+)-3-(2-amino-benzooxazol-5-yl)-1-((S)-2-methyl-2-tetrahydro-furan-2-yl-propyl)-1H-pyrazolo[3,4 -d]pyrimidine-4,6-diamine;
• 3-(2-amino-benzooxazol-5-yl)-1-(2,2-dimethyl-but-3-ynyl)-1H-pyrazolo[3,4-d]pyrimidine-4,6-diamine;
• 3-[4,6-diamino-3-(2-amino-benzooxazol-5-yl)-pyrazolo[3,4-d]pyrimidin-1-yl]-2,2-dimethyl-propan-1-ol ;
• 3-(2-amino-benzooxazol-5-yl)-1-(2-methyl-2-methylsulfanyl-propyl)-1H-pyrazolo[3,4-d]pyrimidine-4,6-diamine;
• 3-(2-amino-4-chloro-benzooxazol-5-yl)-1-(2,2-dimethyl-propyl)-1H-pyrazolo[3,4-d]pyrimidine-4,6-diamine;
• 3-(2-amino-6-methoxy-benzooxazol-5-yl)-1-(2,2-dimethyl-propyl)-1H-pyrazolo[3,4-d]pyrimidine-4,6-diamine;
• 2-[4,6-diamino-1-(2,2-dimethyl-propyl)-1H-pyrazolo[3,4-d]pyrimidin-3-yl]-1H-pyrrolo[2,3-b]pyridin-5-ol;
• 3-(2-amino-6-methoxy-benzooxazol-5-yl)-1-(2,2-dimethyl-butyl)-1H-pyrazolo[3,4-d]pyrimidine-4,6-diamine;
• 3-(2-amino-benzooxazol-5-yl)-1-isopropyl-1H-pyrazolo[3,4-d]pyrimidine-4,6-diamine;
• 3-(2-amino-benzooxazol-5-yl)-1-(2-ethyl-2-methanesulfonyl-butyl)-1H-pyrazolo[3,4-d]pyrimidine-4,6-diamine;
• 3-(2-amino-benzooxazol-5-yl)-4-chloro-1-isobutyl-1H-pyrazolo[3,4-d]pyrimidin-6-ylamine;
• 3-(2-amino-benzooxazol-5-yl)-1-isopropyl-1H-pyrazolo[3,4-d]pyrimidin-6-ylamine;
• 3-(2-amino-6-chloro-benzooxazol-5-yl)-1-((S)-1,2-dimethyl-propyl)-1H-pyrazolo[3,4-d]pyrimidine-4,6-diamine;
• 3-(2-Amino-benzooxazol-5-yl)-1-isopropyl-N*6*-methyl-1H-pyrazolo[3,4-d]pyrimidine-4,6-diamine;
• 5-(6-amino-4-methyl-1-neopentyl-1H-pyrazolo[3,4-d]pyrimidin-3-yl)benzo[d]oxazol-2-amine;
• 3-(2-cyclopropylamino-benzooxazol-5-yl)-1-isopropyl-1H-pyrazolo[3,4-d]pyrimidine-4,6-diamine;
• 3-(2-amino-7-methyl-benzooxazol-5-yl)-1-(2,2-dimethyl-butyl)-1H-pyrazolo[3,4-d]pyrimidine-4,6-diamine;
• 3-(2-amino-benzooxazol-5-yl)-1-(1-methyl-cyclobutylmethyl)-1H-pyrazolo[3,4-d]pyrimidine-4,6-diamine;
• 5-(6-Amino-4-chloro-1-isopropyl-1H-pyrazolo[3,4-d]pyrimidin-3-yl)benzo[d]oxazol-2-amine;
or a pharmaceutically acceptable salt thereof.

7. Composition comprising, in a physiologically acceptable medium, a compound according to one of claims 1 to 6 or a pharmaceutically acceptable salt thereof.

8. Composition according to claim 7, **characterized in that** it comprises between 0.001% and 5% by weight of said compound based on the total weight of the composition.

9. Composition according to claim 7 or 8, **characterized in that** it is in a form suitable for oral or topical administration.

10. Composition according to claim 9, **characterized in that** it is in a form suitable for topical administration.

11. Composition according to one of claims 7 to 10, for use as a medicament.

12. Composition according to claim 11, for use in the treatment of dermatological conditions linked to a keratinization disorder having a proliferative, inflammatory and/or immuno-allergic component such as psoriasis, atopic dermatitis, actinic keratosis, or acne.

13. Composition according to claim 12, for use in the treatment of atopic dermatitis.

14. Composition according to claim 13, for use in the treatment of the inflammatory component in atopic dermatitis.

15. Composition according to claim 13, for use in reinforcing the skin barrier function in a patient suffering from atopic dermatitis.
